(19) Europäisches Patentamt
European Patent Office
Office européen des brevets

(11) Veröffentlichungsnummer: **0 185 390 B1**

# (12) EUROPÄISCHE PATENTSCHRIFT

(45) Veröffentlichungstag der Patentschrift: **02.10.91**

(51) Int. Cl.$^5$: **C07K 5/08**, C07K 5/06, A61K 37/02

(21) Anmeldenummer: **85116383.2**

(22) Anmeldetag: **23.12.85**

## (54) Tripeptidyl-argininaldehyde, Verfahren zu ihrer Herstellung und diese enthaltende Arzneimittel sowie N-(Monoalkyl)- und N,N-Di-(alkyl)-Xxx-L-prolin-Dipeptide.

(30) Priorität: **21.12.84 HU 476384**

(43) Veröffentlichungstag der Anmeldung:
**25.06.86 Patentblatt 86/26**

(45) Bekanntmachung des Hinweises auf die Patenterteilung:
**02.10.91 Patentblatt 91/40**

(84) Benannte Vertragsstaaten:
**AT BE CH DE FR GB IT LI LU NL SE**

(56) Entgegenhaltungen:
**EP-A- 0 019 589**
**EP-A- 0 110 306**
**DE-A- 3 000 225**
**GB-A- 2 091 270**
**US-A- 4 434 096**
**US-A- 4 448 717**

(73) Patentinhaber: **RICHTER GEDEON VEGYESZETI GYAR R.T.**
**Gyömröi ut 19-21**
**H-1475 Budapest X(HU)**

(72) Erfinder: **Bajusz, Sándor, Dr.**
**Derék u. 16/a**
**H-1016 Budapest(HU)**
Erfinder: **Széll geb. Hasenöhrl, Erzsébet**
**Heves u. 64**
**H-1106 Budapest(HU)**
Erfinder: **Bagdy, Dániel, Dr.**
**Promontor u. 12**
**H-1222 Budapest(HU)**
Erfinder: **Barabás, Eva, Dr.**
**Pusztaszeri u. 6**
**H-1025 Budapest(HU)**
Erfinder: **Di szegi, Mariann**
**Eperjesi u. 3**
**H-2143 Kerepestarcsa(HU)**
Erfinder: **Fittler, Zsuzsa**
**Szentkirályi u. 35**
**H-1088 Budapest(HU)**

EP 0 185 390 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **J zsa, Ferencz**
**Murányi u. 14/a**
**H-1192 Budapest(HU)**
Erfinder: **Horváth, Gyula, Dr.**
**Kigy u. 2-4**
**H-1052 Budapest(HU)**
Erfinder: **Tomori geb. Joszt, Eva, Dr.**
**Berda J. u. 44**
**H-1043 Budapest(HU)**

(74) Vertreter: **Beszédes, Stephan G., Dr. Patentanwalt**
**Münchener Strasse 80a Postfach 1168**
**W-8060 Dachau(DE)**

## Beschreibung

Die Erfindung betrifft neue Tripeptidyl-aldehyde, ein Verfahren zur Herstellung derselben und diese Verbindungen enthaltende Arzneimittel, insbesondere solche mit die Blutgerinnung hemmender Wirkung, sowie neue Zwischenprodukte darstellende N-(Monoalkyl)- und N,N-Di-(alkyl)-Xxx-L-prolin-Dipeptide.

Es ist bekannt, daß sowohl die Blutgerinnung als auch die Lösung des Blutgerinnsels von proteolytischen Reaktionen hervorgerufen werden. Beim Vorgang der Blutgerinnung selbst kann die Thrombin-Fibrinogen-Reaktion, bei welcher das im Blutplasma gelöste Fibrinogen zum unlöslichen Fibrinpolymer umgesetzt wird, als Schlüsselreaktion betrachtet werden. Beim lytischen Vorgang tritt dagegen die proteolytische Wirkung des Plasmines in den Vordergrund, wobei das Fibrinpolymer in lösliche Fragmente gespalten wird. Von einem therapeutisch einsetzbaren die Blutgerinnung hemmenden Mittel wird erwartet, daß es im Laufe der Blutgerinnungshemmung die Lösung des Blutgerinnsels selbst, also die Plasmin-Fibrin-Reaktion, nicht hemmt. Freie Tripeptid-aldehyd-Salze der Struktur D-Phenylalanyl-L-prolyl-L-argininaldehyd (ungarische Patentschrift 169 870 und belgische Patentschrift 891 708) entsprechen dieser Erwartung, indem sie keinen Einfluß auf die Plasmin-Fibrin-Reaktion ausüben. In ihrer Gegenwart wird ie nach der Konzentration entweder überhaupt kein Gerinnsel oder nur ein loses Gerinnsel gebildet, das leicht vom Plasmin gelöst werden kann. Es ist aber auch bekannt, daß Tripeptid-aldehyde mit freier endständiger Aminogruppe bei gleichzeitigem Verlust ihrer hohen enzymhemmenden Aktivität in der Regel sich leicht zersetzen, allein das D-Phenylalanyl-L-prolyl-L-argininaldehyd-sulfat der belgischen Patentschrift 891 708 hat eine ausreichende Stabilität, indem es bei 5°C in Lösung seine Ausgangsaktivität für lange Zeit bewahrt.

Stabilitätsuntersuchungen des D-Phenylalanyl-L-prolyl-L-argininaldehyd-sulfats, die in wäßriger Lösung bei höheren Temperaturen durchgeführt wurden, zeigten, daß dieses Peptid bei 80 bis 100°C innerhalb einiger Stunden und bei 37 bis 40°C während 10 bis 14 Tage praktisch vollständig zu einer stabilen tricyclischen Verbindung ohne enzymhemmende Wirkung, deren Struktur durch Massen- und Kernresonanzspektren bewiesen wurde [1,2,4,5,6a-Hexahydro-1-benzyl-2-oxo-8-(4'-guanidino)-butyl-pyrrolo[1,2-a]-imidazolo[2,1-c]pyrazin], umgesetzt wird. Ferner wurde noch festgestellt, daß bei bestimmten Acyl-tripeptidylaldehyden keine obige irreversible Umsetzung erfolgt. Diese Acyl-tripeptidyl-aldehyde, wie tert.-Butoxycarbonyl-D-phenylalanyl-L-prolyl-L-argininaldehyd-hemisulfat, hemmen dagegen neben der Thrombin-Fibrinogen-Reaktion zusätzlich auch noch die Plasmin-Fibrin-Reaktion erheblich [belgische Patentschrift 891 708 und S. Bajusz und Mitarbeiter: Peptides, Synthesis-Structure-Function (Eds. H. D. Rich und E. Gross), Pierce Chem. Co., Rockford, Illinois, USA, Seite 817].

Der Erfindung liegt die Aufgabe zugrunde, unter Behebung der Nachteile des Standes der Technik neue Tripeptidyl-aldehyde mit überlegenen pharmakologischen Wirkungen, vor allem der selektiven die Blutgerinnung hemmenden Eigenschaft des bekannten D-Phenylalanyl-L-prolyl-L-argininaldehyd-sulfates, jedoch höherer Stabilität als die des letzteren und ohne Neigung zur irreversiblen Cyclisation, ein Verfahren zu ihrer Herstellung und diese Verbindungen enthaltende Arzneimittel sowie neue Zwischenprodukte für ihre Herstellung zu schaffen.

Diese Aufgabe wurde überraschenderweise durch die Erfindung erreicht.

Es wurde nämlich überraschenderweise festgestellt, daß bei Tripeptidyl-aldehyden, welche an der endständigen Aminogruppe alkyliert sind, zum Beispiel N-Methyl-, N-Äthyl- oder N-Isobutyl-D-phenylalanyl-L-prolyl-L-argininaldehyd-sulfat, die bei D-Phenylalanyl-L-prolyl-L-argininaldehyd-sulfat auftretende irreversible Umwandlung, also die Bildung der oben erwähnten oder einer ähnlichen tricyclischen Verbindung, nicht vor sich geht. Bei den Untersuchungen wurden das D-Phenylalanyl-L-prolyl-L-argininaldehyd-sulfat sowie die entsprechenden N-(Alkyl)-derivate in wäßriger Lösung (10 mg/ml, pH-Wert: 6) 5 Tage lang bei 40°C aufbewahrt. Die vor sich gehende Strukturumwandlung wurde mittels Chromatographie und Bestimmung der Antithrombinaktivität geprüft.

Die im Lösungsmittelgemisch von Äthylacetat, Pyridin, Essigsäure und Wasser im Volumverhältnis von 30 : 20 : 6 : 11 durchgeführte Silicagel-Dünnschichtchromatographie zeigt eindeutig die stufenweise Umwandlung von D-Phenylalanyl-L-prolyl-L-argininaldehyd-sulfat: auf Kosten des Tripeptid-Flecks ($R_F$-Wert = 0,40) wird das Fleck der tricyclischen Verbindung ($R_F$-Wert = 0,60) immer intensiver, während im Dünnschichtchromatogramm der entsprechenden N-(Methyl)-, N-(Äthyl)- und N-(Isobutyl)- sowie N,N-Di-(methyl)-Verbindungen nur die Peptidflecke selbst ($R_F$-Werte = 0,43, 0,50, 0,64 und 0,37) sichtbar waren, da dort keine obige Umwandlung vor sich geht.

Es ist noch hervorzuheben, daß andere Salze der N-(Alkyl)-tripeptidyl-aldehyde beziehungsweise N,N-Di-(alkyl)-tripeptidyl-aldehyde, zum Beispiel ihre Acetate, gleichfalls nicht in die entsprechenden tricyclischen Verbindungen überführt werden, während das D-Phenylalanyl-L-prolyl-L-argininaldehyd-acetat besonders zu einer solchen Umwandlung neigt.

Gegenstand der Erfindung sind daher Tripeptidylaldehyde der allgemeinen Formel

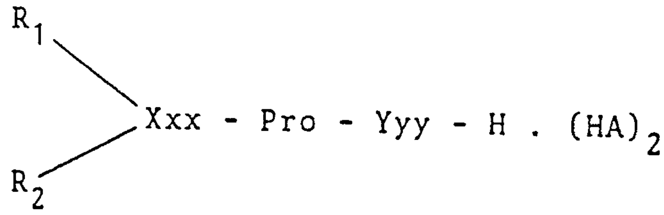

I ,

worin

R₁ für ein Wasserstoffatom oder einen Alkylrest mit 1 bis 6 Kohlenstoffatom(en) steht,
R₂ einen Alkylrest mit 1 bis 6 Kohlenstoffatom(en) bedeutet,
Xxx einen Rest von D-Phenylalanin oder einen Rest einer D-α-Aminosäure, die in der Seitenkette einen Alkylrest mit 1 bis 4 Kohlenstoffatom(en) hat, darstellt,
Pro für einen Rest von L-Prolin steht,
Yyy einen Rest von L-, D- oder DL-Arginin bedeutet und
A einen Acylrest darstellt,

wobei $R_1$ und $R_2$ an die Aminogruppe des Restes der Aminosäure, für die Xxx stehen kann, gebunden ist.

Die britische Offfenlegungsschrift 2 091 270 und die deutsche Offenlegungsschrift 30 00 225 beschreiben D-Phenylalanyl-L-prolyl-Largininaldehydsulfat beziehungsweise das entsprechende $N^G$-Carboxylderivat mit einer die Blutgerinnung hemmenden Wirkung.

Die US-Patentschrift 4 448 717 beschreibt Tetrapeptidylmethyl- oder -ethylketone mit einer freien Aminoendgruppe, die eine analgetische und neurologische Aktivität besitzen.

Die US-Patentschrift 4 434 096 beschreibt Tripeptidthiolester, welche zur quantitativen Bestimmung von proteolytischen Enzymen in biologischen Flüssigkeiten eingesetzt werden.

Die europäische Offenlegungsschrift 0 110 306 beschreibt chromogene, Aminosäuren enthaltende Verbindungen, die als Substrate zum Nachweis und zur quantitativen Bestimmung von hydrolytisch wirkenden Enzymen verwendet werden.

Die europäische Offenlgungsschrift 0 019 589 betrifft Tripeptidderivate, die zur Bestimmung von proteolytischen Enzymen eingesetzt werden.

Vorzugsweise ist beziehungsweise sind der beziehungsweise die Alkylrest(e), für den beziehungsweise die $R_1$ und/oder $R_2$ steht beziehungsweise stehen kann beziehungsweise können, [ein] solche [r] mit 1 bis 4, insbesondere 1 oder 2, ganz besonders 1, Kohlenstoffatom(en).

Ferner ist es bevorzugt, daß Xxx einen Rest von D-Phenylalanin darstellt.

Vorzugsweise ist der Alkylrest der Seitenkette des Restes der D-α-Aminosäure, den Xxx darstellen kann, ein solcher mit 3 oder 4, insbesondere 4, Kohlenstoffatomen, wobei für Xxx als Rest der D-α-Aminosäure ein solcher von D-Alloisoleucin, D-Isoleucin, D-Norleucin, D-Valin oder D-Norvalin besonders bevorzugt ist, ganz besonders der erstere.

Es ist auch bevorzugt, daß Yyy einen Rest von L-Arginin bedeutet.

Der Acylrest, für den A steht, kann ein Mineralsäurerest, vorzugsweise Salzsäure-, Schwefelsäure-, Phosphorsäure-, oder Salpetersäurerest, oder ein organischer Säurerest, vorzugsweise Weinsäure-, Essigsäure-, Citronensäure-, Apfelsäure-, Milchsäure-, Fumarsäure-, Benzoesäure-, Glykolsäure-, Gluconsäure-, Gulonsäure-, Bernsteinsäure- oder Arylsulfonsäurerest, sein.

Ganz besonders bevorzugte erfindungsgemäße Tripeptidyl-aldehyde sind N-(Methyl)-D-phenylalanyl-L-prolyl-L-argininaldehyd-sulfat, N,N-Di-(methyl)-D-phenylalanyl-L-prolyl-L-argininaldehyd-sulfat, N-(Äthyl)-D-phenylalanyl-L-prolyl-L-argininaldehyd-sulfat, N-(Isobutyl)-D-phenylalanyl-L-prolyl-L-argininaldehyd-sulfat, N-(Methyl)-D-alloisoleucyl-L-prolyl-L-argininaldehyd-sulfat und N-(n-Hexyl)-D-phenylalanyl-L-prolyl-L-argininaldehyd-sulfat.

Ferner wurde festgestellt, daß die erfindungsgemäßen Tripeptidyl-aldehyde der allgemeinen Formel I durch Acylieren des an der Guanidinogruppe geschützten Argininlactames mit dem endständigen Aminodipeptidrest als Säurekomponente, Reduzieren des entstandenen geschützten Tripeptidyl-lactames zum entsprechenden geschützten Tripeptidyl-aldehyd und nach Entfernen der Schutzgruppe(n) erfolgendes Isolieren des erfindungsgemäßen N-(Alkyl)- beziehungsweise N,N-Di-(alkyl)-tripeptidyl-aldehydes in Salzform hergestellt werden können.

Gegenstand der Erfindung ist daher auch ein Verfahren zur Herstellung der erfindungsgemäßen Verbindungen, welcher dadurch gekennzeichnet ist, daß das an der Guanidinogruppe geschützte Argininlactam mittels einer in der Peptidchemie bekannten Verfahrensweise mit einem an seiner endständigen

Aminogruppe eine Schutzgruppe aufweisenden N-(Monoalkyl) -Xxx-L-prolin-Dipeptid oder einem N,N-Di-(alkyl)-Xxx-L-prolin-Dipeptid der allgemeinen Formel

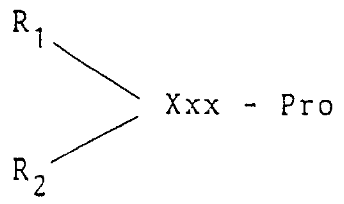

II ,

worin $R_1$ , $R_2$ und Xxx die oben angegebenen Bedeutungen hat. kondensiert wird, das erhaltene geschützte Tripeptidyl-argininlactam zum entsprechenden geschützten Tripeptidyl-aldehyd reduziert wird und nach Abspalten der Schutzgruppe(n) den N-(Alkyl)-beziehungsweise N,N-Di-(alkyl)-tripeptidyl-aldehyd in Salzform isoliert wird.

Nach einer vorteilhaften Ausführungsform des erfindungsgemäßen Verfahrens unter Einbeziehung der Ausgangssubstanzherstellung wird das N-(Benzyloxycarbonyl)-derivat des D-Phenylalanyl-L-prolins in Gegenwart von Natriumhydrid mit Methyljodid methyliert und das gebildete N-(Benzyloxycarbonyl)-N-(methyl)-D-phenylalanyl-L-prolin durch ein gemischtes Anhydrid an das N$^{\omega}$-Benzyloxycarbonyl)-L-argininlactam gebunden. Das entstandene geschützte Tripeptidyl-argininlactam wird mit Lithiumaluminiumhydrid zu N-(Benzyloxycarbonyl)-N-(methyl)-D-phenylalanyl-L-prolyl-N$^{\omega}$-(benzyloxycarbonyl)-L-argininaldehyd reduziert, die Schutzgruppen werden in Gegenwart von Schwefelsäure mittels Hydrogenolyse entfernt und das gebildete N-(Methyl)-D-phenylalanyl-L-prolyl-L-argininaldehyd-sulfat wird isoliert.

Nach einer weiteren vorteilhaften Ausführungsform des erfindungsgemäßen Verfahrens unter Einbeziehung der Ausgangssubstanzherstellung wird D-Phenylalanyl-L-prolin mit Formaldehyd hydrierend umgesetzt. Das erhaltene N,N-Di-(methyl)-D-phenylalanyl-L-prolin wird durch ein gemischtes Anhydrid mit N$^{\omega}$-(Benzyloxycarbonyl)-L-argininlactam gekoppelt, das gebildete geschützte Tripeptidyl-argininlactam wird mittels Lithium-aluminium-hydrid zu N,N-Di-(methyl)-D-phenylalanyl-L-prolyl-N$^{\omega}$-(benzyloxycarbonyl)-L-argininaldehyd reduziert, die Schutzgruppe wird in Gegenwart von Schwefelsäure mittels Hydrogenolyse abgespalten und das erhaltene N,N-Di-(methyl)-D-phenylalanyl-L-prolyl-L-argininaldehyd-sulfat wird isoliert.

Nach einer noch weiteren vorteilhaften Ausführungsform des erfindungsgemäßen Verfahrens unter Einbeziehung der Ausgangssubstanzherstellung wird D-Phenylalanin mit Acetaldehyd hydrierend umgesetzt, das erhaltene N-(Äthyl)-D-phenylalanin wird mit der Benzyloxycarbonyl-Schutzgruppe versehen, dann zum 2,4,5-Trichlorphenylester umgesetzt und nachfolgend mit L-Prolin kondensiert. Das Reaktionsprodukt N-(Benzyloxycarbonyl)-N-(äthyl)-D-phenylalanyl-L-prolin wird durch ein gemischtes Anhydrid mit N$^{\omega}$-(Benzyloxycarbonyl)-L-argininlactam gekoppelt. Das geschützte Tripeptidyl-lactam wird mit Lithium-aluminium-hydrid zu N-(Benzyloxycarbonyl)-N-(äthyl)-D-phenylalanyl-L-prolyl-N$^{\omega}$-(benzyloxycarbonyl)-L-argininaldehyd reduziert, dann werden die Schutzgruppen in Gegenwart von Schwefelsäure mittels Hydrogenolyse abgespalten und das erhaltene N-(Äthyl)-D-phenylalanyl-L-prolyl-L-argininaldehyd-sulfat wird isoliert.

Die Produkte, die durch das erfindungsgemäße Verfahren erhalten werden, haben D,L,L-Konfiguration, können aber gegebenenfalls bestimmte Mengen eines Produktes mit D,L,D-Konfiguration enthalten, was aber die therapeutische Anwendung des Präparates nicht beeinträchtigt.

Ferner sind Gegenstand der Erfindung Arzneimittel, welche 1 oder mehr erfindungsgemäße Verbindung(en) als Wirkstoff(e), zweckmäßig zusammen mit 1 oder mehr pharmazeutisch üblichen Konfektionierungsmittel(n) enthalten.

Die erfindungsgemäßen Verbindungen haben nämlich wie bereits gesagt überlegene pharmakologische, insbesondere die Blutgerinnung hemmende, Wirkungen.

In eigenen Untersuchungen wurden die Peptide in einer Konzentration von 10 mg/cm$^3$ im Wasser (pH 6,0) gelöst und die Lösungen wurden bei einer Temperatur von 40°C gelagert. Die Änderung der Antithrombin-Aktivität der gelösten Stoffe wurde 5 Tage lang beobachtet. Die Aktivitätswerte wurden in einem System, das aus folgenden Bestandteilen bestand, festgestellt:

- 0,2 cm$^3$ 0,5 %-iges Rinderfibrinogen in 0,9 %-iger Kochsalzlösung,
- 0,1 cm$^3$ einer Tris-(hydroxymethyl)-aminomethan/Chlorwasserstoff-Pufferlösung (pH-Wert 7,2), welche auch die zu untersuchende Peptidlösung enthielt, und
- 0,1 cm$^3$ 5 Einheiten/cm$^3$ einer Lösung von menschlichem Thrombin [US Standard Human Thrombin (NIH, Bethesda, Maryland, USA)].

Die Gerinnungszeit dieses Systems ohne Peptidzusatz betrug im Schnither-Gross Coagulometer 15 Sekunden.

Die blutgerinnungshemmenden Anfangsaktivitäten der einzelnen Tripeptidaldehyd-sulfate sind durch diejenige Molkonzentration gekennzeichnet, welche im Vergleich zur Kontrolle eine fünffache Verlängerung der Gerinnungszeit hervorruft ($I_{80}$-Wert).

Die am ersten, dritten und fünften Tag gemessene Aktivität der einzelnen Peptide wird als Prozent der Anfangsaktivität (O. Tag) angegeben und in Tabelle 1 zusammengefasst.

Tabelle 1

Änderung der Antithrombin-Aktivität von Tripeptidaldehyd-sulfaten in wässriger Lösung, bei pH 6,0 und 40°C

| Peptid-aldehyd | Anfangsaktivität $I_{80}$, µM* | 0. Tag | Aktivität (%) 1. Tag | 3. Tag | 5. Tag |
|---|---|---|---|---|---|
| N-Methyl-D-phenylalanyl-L-prolyl-L-argininaldehyd-sulfat | 0,25 | 100 % | 100 | 100 | 100 |
| N-Äthyl-D-phenylalanyl-L-prolyl-L-argininaldehyd-sulfat | 0,21 | 100 % | 100 | 100 | 85 |
| N-Isobutyl-D-phenylalanyl-L-prolyl-L-argininaldehyd-sulfat | 0,31 | 100 % | 100 | 85 | 80 |
| D-Phenylalanyl-L-prolyl-L-argininaldehyd-sulfat (Referenzsubstanz) | 0,25 | 100 % | 100 | 50 | 40 |

* Peptid-Konzentration des Reaktionsgemisches in µM, die im Vergleich zur Kontrolle eine fünffache Verlängerung der Gerinnungszeit hervorruft

Während der Beobachtungsperiode wurde die Anfangsaktivität von D-Phenylalanyl-L-prolyl-L-argininaldehyd-sulfat um die Hälfte verringert, während das N-Methyl-Derivat seine ursprüngliche Aktivität behielt, und auch die Aktivität der N-Äthyl- und N-Isobutyl-Verbindung nur um 15 - 20 % verringert wurde. Unter den Versuchsbedingungen kann auch mit einer partiellen Racemisierung des endständigen L-Argininaldehyds gerechnet werden, womit der geringe Aktivitätsverlust der N-Äthyl- bzw. N-Isobutyl-Derivate erklärt werden kann.

Die Wirkung der N-Alkyl-tripeptidaldehyde auf die Plasmin-Fibrin Reaktion wurde auch untersucht und am Beispiel des N-Methyl-D-phenylalanyl-L-prolyl-L-argininaldehyd-sulfats im folgenden System ermittelt:

| | |
|---|---|
| 0.2 $cm^3$ | 0,5 %-iges Rinderfibrinogen in 0,9 %-iger Kochsalzlösung, |
| 0,1 $cm^3$ | einer Tris-(hydroxymethyl)-aminomethan/Chlorwasserstoff-Pufferlösung (pH-Wert 7,2), welche auch das zu untersuchende Peptid enthielt, und |
| 0,1 $cm^3$ | einer Lösung von menschlichem Thrombin [US Standard Human Thrombin (NIH, Bethesda, Maryland, USA], 10 Einheiten/$cm^3$, |
| 0,1 Einheit | Plasmin in 0,1 ml Pufferlösung (KABI, Stockholm, Schweden). |

Die Gerinnungszeit des im System gebildeten Fibringels ohne Peptidzusatz beträgt 10 Minuten.

Als Kontrollverbindung wurden D-Phenylalanyl-L-prolyl-L-argininaldehyd-sulfat sowie das oben erwähnte tert.-Butoxycarbonyl-D-phenylalanyl-L-prolyl-L-argininaldehyd-hemisulfat eingesetzt. Die Ergebnisse wurden in Tabelle 2 zusammengefasst.

Tabelle 2

Wirkung der Tripeptidaldehyde auf die Plasmin-Fibrin-Reaktion

| Peptidaldehyd | Peptidgehalt µM | Lösungszeit des Fibringerinnsels Minute* |
|---|---|---|
| N-Methyl-D-phenylalanyl-L-prolyl-L-argininaldehyd-sulfat | 0,18 | 10 |
| | 0,36 | ** |
| | 0,72 | kein Gerinnsel |
| D-Phenylalanyl-L-prolyl-L-argininaldehyd-sulfat | 0,18 | 10 |
| (Referenzsubstanz) | 0,36 | ** |
| | 0,72 | kein Gerinnsel |
| tert.-Butoxycarbonyl-D-phenylalanyl-L-prolyl-L-argininaldehyd-hemisulfat | 0,18 | 10 |
| (Referenzsubstanz) | 0,36 | 20 |
| | 0,72 | 40 |

* Lösungszeit des Fibringerinnsels ohne Peptidzusatz 10 Minuten

** Keine messbare Lösungszeit, Gerinnsel loser Struktur

Aus der obigen Tabelle geht hervor, dass der N-Alkyl-tripeptidaldehyd sich wie das eine freie endständige Aminogruppe aufweisende Analoge verhält. Bei kleinen Mengen löst sich das entstandene Gerinnsel innerhalb der Kontrollzeit, bei grösseren Mengen wird entweder überhaupt kein Gerinnsel gebildet, oder nur eines mit loser Gelstruktur, dagegen verlängert das Acyl-tripeptidaldehyd-hemisulfat konzentrationsabhängig die Plasmin-Hydrolysezeit des Fibringerinnsels.

Aus obigen Untersuchungen geht hervor, dass die neuen, an der endständigen Aminogruppe alkylierten Tripeptidaldehyde wesentlich stabiler sind als das bekannte D-Phenylalanyl-L-prolyl-L-argininaldehyd-sulfat, gleichzeitig aber ähnlich selektive Gerinnungshemmer sind, indem sie bei wirksamer Hemmung der Thrombin-Fibrinogen-Reaktion den Ablauf der Plasmin-Fibrin Reaktion nicht beeinflussen.

Die neuen N-Alkyl-tripeptidaldehyde der allgemeinen Formel I besitzen auch in vivo signifikante

blutgerinnungshemmende Aktivität. In eigenen Untersuchungen wurden die Peptide New Zealand Hasen (Gewicht: 2 - 3 kg) intravenös bzw. peroral in physiologischer Kochsalzlösung verabreicht. Bei intravenöser Gabe wurde drei Stunden lang eine Infusion mit Dosen von 0,5 - 2,0 mg/kg/Stunde bei einer Geschwindigkeit von 6 ml/Stunde verabreicht, peroral wurden 20 - 30 mg/kg Peptide, in 2 ml Volumen gelöst, gegeben. Nach der Gabe wurden in 30 Minuten Perioden Blutproben aus der Schwanzvene genommen und die Gerinnungszeit des ganzen Blutes im Thromboelastograph bestimmt [H. Harter: Zeitschrift für klinische Medizin 153, 423 (1955)], gleichzeitig wurde auch die Plasma-Thrombinzeit gemessen [R. T. S. Jim: J Lab. Clin. Med. 50, 45 (1957)]. Die für die therapeutische Wirkung nötigen Mindestdosen (M. Verstraete und R. Verwilghen in Drug Treatment, 2. Aufl., Ed.: G. S. Avery, Churchill Livingstone, Edinburgh-London, 1980, S. 919) wurden in Tabelle 3 zusammengefasst, als Referenzsubstanz wurde D-Phenylalanyl-L-prolyl-L-argininaldehyd-sulfat angewandt.

Die Daten zeigen, dass die N-Alkyl-tripeptidaldehyde die gleiche oder ähnliche Wirksamkeit besitzen, wie die Referenzsubstanz.

Auf Grund der an Hasen durchgeführten intravenösen Infusionsuntersuchungen wurde die Humandosis der intravenösen Infusion zu 1 - 2 mg/kg/Stunde festgesetzt.

Tabelle 3

In vivo gerinnungshemmende Wirkung von N-Alkyl-tripeptidaldehyden in männlichen New Zealand Hasen

| Tripeptidaldehyd | Kleinste wirksame Dosis intravenös mg/kg/Stunde | peroral mg/kg |
|---|---|---|
| N-Methyl-D-phenylalanyl-L-prolyl-L-argininaldehyd-sulfat | 0,5 | 20 |
| N-Äthyl-D-phenylalanyl-L-prolyl-L-argininaldehyd-sulfat | 0,5 | 20 |
| N-Isobutyl-D-phenylalanyl-L-prolyl-L-argininaldehyd-sulfat | 0,5 | 20 |
| N,N-Dimethyl-D-phenylalanyl-L-prolyl-L-argininaldehyd-sulfat | 2,0 | 30 |
| N-Methyl-D-alloisoleucyl-L-prolyl-L-argininaldehyd-sulfat | 1,0 | 30 |
| D-Phenylalanyl-L-prolyl-L-argininaldehyd-sulfat (Referenzsubstanz) | 0,5 | 20 |

Die im erfindungsgemäßen Verfahren als Ausgangssubstanzen verwendbaren N-(Monoalkyl)-Xxx-L-prolin-Dipeptide beziehungsweise N,N-Di-(alkyl)-Xxx-L-prolin-Dipeptide der allgemeinen Formel II sind ebenfalls neue Verbindungen.

Gegenstand der Erfindung sind daher auch N-(Monoalkyl)- und N,N-Di-(alkyl)-Xxx-L-prolin-Dipeptide der allgemeinen Formel

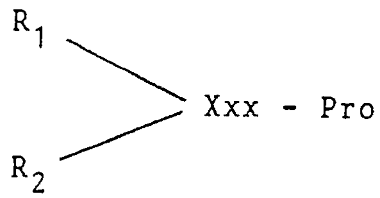

II,

worin

$R_1$ für ein Wasserstoffatom oder einen Alkylrest mit 1 bis 6 Kohlenstoffatom(en) steht,

$R_2$ einen Alkylrest mit 1 bis 6 Kohlenstoffatom(en) bedeutet und

Xxx einen Rest von D-Phenylalanin oder einen Rest einer D-$\alpha$-Aminosäure, die in der Seitenkette einen Alkylrest mit 1 bis 4 Kohlenstoffatom(en) hat, darstellt.

Die Eigenschaften dieser neuen Verbindungen sind die Ursache für die überlegenen pharmakologischen Wirkungen der aus ihnen herstellbaren erfindungsgemäßen Tripeptidyl-aldehyde der allgemeinen Formel I.

Die erfindungsgemäßen N-(Monoalkyl)- und N,N-Di-(alkyl)-Xxx-L-prolin-Dipeptide der allgemeinen Formel II können wie folgt hergestellt werden.

Die an der endständigen Aminogruppe geschützte Aminosäure, deren Rest Xxx ist, wird gemäß einer in der Peptidchemie bekannten Verfahrensweise mit L-Prolin kondensiert und das erhaltene geschützte Dipeptid wird alkyliert oder

ein Xxx-L-prolin-Dipeptid mit endständiger freier Aminogruppe wird entweder mono- oder dialkyliert beziehungsweise eine Aminosäure, deren Rest Xxx ist und welche entweder monoalkyliert und geschützt oder dialkyliert ist, wird mittels einer in der Peptidchemie bekannten Verfahrensweise mit L-Prolin kondensiert.

Die Erfindung wird an Hand der folgenden Beispiele näher erläutert. Die $R_F$-Werte wurden durch Dünnschichtchromatographie an Silicagel (Kieselgel G, Reanal, Budapest) in folgenden Lösungsmittelgemischen bestimmt:

1. Äthylacetat-Pyridin-Essigsäure-Wasser (960:20:6:11)
2. Äthylacetat-Pyridin-Essigsäure-Wasser (480:20:6:11)
3. Äthylacetat-Pyridin-Essigsäure-Wasser (240:20:6:11)
4. Äthylacetat-Pyridin-Essigsäure-Wasser (120:20:6:11)
5. Äthylacetat-Pyridin-Essigsäure-Wasser (60:20:6:11)
6. Äthylacetat-Pyridin-Essigsäure-Wasser (30:20:6:11)

Die oben und in den Beispielen angegebenen Verhältnisse sind Volumverhältnisse.

## Beispiel 1

### N-Methyl-D-phenylalanyl-L-prolyl-L-argininaldehyde-sulfat

Stufe 1: N-Benzyloxycarbonyl-N-methyl-D-phenylalanyl-L-prolyl-$N^{\omega}$-benzyloxycarbonyl-L-arginin-lactam

42,95 g (0,11 Mol) $N^{\alpha}$-t-Butyloxycarbonyl-$N^{\omega}$-benzyloxycarbonyl-L-arginin-lactam wird in 110 ml wasserfreiem Chloroform suspendiert und unter Rühren mit 275 ml salzsaurem Äthylacetat (Konzentration: 0,11 - 0,15 g/ml) versetzt. Nach dreistündigem Rühren wird das Reaktionsgemisch mit 400 ml Diäthyläther verdünnt, die ausgeschiedene Kristallmasse filtriert, zweimal mit 100 ml Diäthyläther und zweimal mit 50 ml Aceton gewaschen, dann im Vacuumexsiccator über Phosphorpentoxid und Kaliumhydroxid getrocknet. Nach ungefähr einstündigem Trocknen wird die Verbindung in 100 ml Dimethylformamid gelöst, die Lösung wird auf -15°C gekühlt und bei dieser Temperatur unter Rühren erst mit 20 ml, dann mit 10 - 15 ml Triäthylamin so versetzt, dass in der Gasphase Basenüberfluss nachweisbar sei. Die Suspension wird zu dem folgenden gemischten Anhydrid gegeben.

50,9 g (0,1 Mol) N-Benzyloxycarbonyl-N-methyl-D-phenylalanyl-L-prolin-Cyclohexylammoniumsalz wird in 200 ml Diäthyläther und 120 ml n Schwefelsäure gelöst. Die diäthylätherische Phase wird dreimal mit je 30 ml Wasser gewaschen, über wasserfreiem Natriumsulfat getrocknet und am Wasserbad bei einer Temperatur von 40°C bei 25 - 30 Millibar eingedampft. Der Rückstand und 11,2 ml (0,1 Mol) N-Methylmorpholin werden in 70 ml Dimethylformamid gelöst, auf -15°C abgekühlt und bei dieser Temperatur mit 13,2 ml (0,1 Mol) Isobutyloxycarbonylchlorid versetzt. Nachfolgend (3 - 5 Minuten) wird noch 5 Minuten lang gerührt, dann wird dem gebildeten gemischten Anhydrid die obige Dimethylformamid-Suspension

zugefügt. Das Reaktionsgemisch wird eine Stunde lang bei -15°C und eine Stunde lang bei 0°C gerührt, dann mit 200 ml Benzol verdünnt, und die ausgeschiedenen Kristalle werden abfiltriert. Die Benzol-Dimethylformamid-Lösung wird mit 150 ml Wasser verdünnt und die Phasen getrennt. Die untere, wässrige Dimethylformamid-Phase wird dreimal mit je 50 ml Benzol extrahiert. Die vereinigten Benzol-Phasen werden dreimal mit 30 ml Wasser gewaschen, über wasserfreiem Natriumsulfat getrocknet und vom Wasserbad bei max. 40°C und 20 - 25 Millibar abgedampft. Der ölige Rückstand wird in 60 ml eines Gemisches von Benzol-Tetrahydrofuran (8:2) gelöst und in dem selben Lösungsmittelsystem an 750 g einer Kieselgel-60-Kolonne chromatographiert. Die Fraktionen werden mittels Silicagel-Dünnschichtchromatographie in einem Lösungsmittelsystem von Äthylacetat-Pyridin-Essigsäure-Wasser (480:20:6:11) analysiert; das Zielprodukt hat einen $R_F$-Wert von 0,70 - 0,76. Die Fraktionen, die das reine Hauptprodukt enthalten, werden vereinigt und am Wasserbad bei max. 40°C, bei 20 - 25 Millibar auf 100 ml Volumen eingeengt. Dieses Konzentrat wird mit 500 ml Petroläther vermischt. Die entstandene Suspension wird filtriert, zweimal mit je 100 ml Petroläther gewaschen und im Vacuumexsiccator über Schwefelsäure und Paraffinspänen getrocknet.
Ausbeute: 45 g (65 %)
$[\alpha]_D^{20}$ = + 13,5° (c = 1, Tetrahydrofuran)
$R_F^2$ = 0,70-0,76

Analyse für $C_{37}H_{42}O_7N_6$ (682,75)

| | | | | | | |
|---|---|---|---|---|---|---|
| berechnet: | C | 65,08 | H | 6,20 | N | 12,31 % |
| gefunden: | C | 65,4 | H | 6,4 | N | 12,1 % |

Stufe 2: N-Benzyloxycarbonyl-N-methyl-D-phenylalanyl-L-prolyl-$N^\omega$-benzyloxycarbonyl-L-argininaldehyd

34,15 g (0,05 Mol) N-Benzyloxycarbonyl-N-methyl-D-phenylalanyl-L-prolyl-$N^\omega$-benzyloxycarbonyl-L-arginin-lactam (Beispiel 1, Stufe 1) werden in 150 ml Tetrahydrofuran gelöst und unter Rühren mit 0,0375 Mol Lithium-aluminium-hydrid in Tetrahydrofuran versetzt. Der Fortschritt der Reduktion wird dünnschichtchromatographisch im Lösungsmittelsystem Äthylacetat-Pyridin-Essigsäure-Wasser (240:20:6:11) kontrolliert ($R_F$-Wert des Lactams bzw. Aldehyds ungefähr 0,8 bzw. 0,5). Falls nötig,sollten weitere Mengen von Lithium-aluminium-hydrid zugefügt werden, dann wird der pH-Wert der Reaktionslösung unter Kühlung und Rühren mit n Schwefelsäure auf 2 eingestellt. Die Lösung wird bis zur Opaleszenz mit Wasser verdünnt (ungefähr 300 ml) und nachfolgend zweimal mit 100 ml Hexan extrahiert. Die wässrige Tetrahydrofuran-Lösung wird zweimal mit 250 ml Methylenchlorid extrahiert, die vereinigten Methylenchlorid-Extrakte zweimal mit je 50 ml Wasser, zweimal mit je 60 ml 5 %-iger Natriumbicarbonat-Lösung und wieder zweimal mit je 50 ml Wasser gewaschen, über wasserfreiem Natriumsulfat getrocknet, dann auf dem Wasserbad bei max. 40°C und 20 - 25 Millibar auf 50 - 60 ml eingeengt. Der Rückstand wird mit 100 ml Benzol versetzt und wie oben eingeengt. Der Rückstand wird mit 100 ml Cyclohexan verdünnt, der ausgeschiedene Niederschlag abfiltriert, zweimal mit 30 ml Cyclohexan gewaschen und im Vakuumexsiccator über Paraffinspänen getrocknet.
Ausbeute: 27 g (70 %) eines Produktes, das gemäss der Elementaranalyse 1 Mol Cyclohexan enthält.
$R_F^3$ = 0,52 - 0,62
$[\alpha]_D^{20}$ = + 16,8° (c = 1, Tetrahydrofuran)

Analyse für $C_{37}H_{44}O_7N_6 \cdot C_6H_{12}$ (768,93)

| | | | | | | |
|---|---|---|---|---|---|---|
| berechnet: | C | 67,16 | H | 7,34 | N | 10,93 % |
| gefunden: | C | 66,6 | H | 7,2 | N | 10,4 % . |

Stufe 3: N-Methyl-D-phenylalanyl-L-prolyl-L-argininaldehyd-sulfat

23,1 g (0,03 Mol) N-Benzyloxycarbonyl-N-methyl-D-phenylalanyl-L-prolyl-N$^{\omega}$-benzyloxycarbonyl-L-argininaldehyd (Beispiel 1, Stufe 2) werden in 150 ml Äthanol gelöst, mit 50 ml entionisiertem Wasser, 48 ml 1 n Schwefelsäure und 3 g 10 %-igem Pd/C-Katalysator versetzt und hydriert. Der Fortschritt der Reaktion wird dünnschichtchromatographisch im Lösungsmittelsystem Äthylacetat-Pyridin-Essigsäure-Wasser (30:20:6:11) kontrolliert ($R_F$-Werte des Ausgangsproduktes, des Zwischenproduktes und des Endproduktes sind ungefähr 0,9, 0,7 bzw. 0,4). Nach beendeter Reaktion wird der Katalysator abfiltriert und dreimal mit je 30 ml entionisiertem Wasser gewaschen. Die vereinigten Filtrat und Waschflüssigkeiten werden im Rotationsverdampfer auf 100 ml eingeengt. Der wässrige Rückstand wird mit entionisiertem Wasser auf ungefähr 200 ml verdünnt. Falls sein pH nicht zwischen 6,0 und 6,5 ist, sollte es mit 0,1 n Schwefelsäure bzw. mit einem Ionenaustauscher im OH -Cyclus (z. B. AG 1X8) nacheingestellt und nachfolgend lyophilisiert werden.
Ausbeute: 12,4 g (75 %)
$R_F^6$ = 0,39 - 0,47
$[\alpha]_D^{20}$ = -131° (c = 1, Wasser)

Analyse für $C_{21}H_{32}O_3N_6 \cdot 7/8(H_2SO_4) \cdot 2,5(H_2O)$ (547,37)

| | | | | | | | | | | |
|---|---|---|---|---|---|---|---|---|---|---|
| berechnet: | C | 46,07 | H | 7,13 | N | 15,35 | S | 5,12 | % | |
| gefunden: | C | 46,4 | H | 7,2 | N | 15,05 | S | 5,0 | % | . |

Die Ausgangsstoffe können nach der folgenden Verfahrensweise hergestellt werden:

N-Benzyloxycarbonyl-N-methyl-D-phenylalanyl-L-prolin

Stufe A: Benzyloxycarbonyl-D-phenylalanin-2,4,5-trichlorphenylester

60,0 g (0,2 Mol) Benzyloxycarbonyl-D-phenylalanin [H. Yajima und K. Kubo: J. Am. Chem. Soc. 87, 2039-2044 (1965)] und 39,4 g (0,2 Mol) 2,4,5-Trichlorphenol werden in 200 ml Tetrahydrofuran gelöst, die Lösung wird auf 5 - 10°Cgekühlt und unter Rühren innerhalb von ungefähr 30 Minuten mit 41,2 g (0,2 Mol) Dicyclohexylcarbodiimid versetzt und nachfolgend 6 Stunden lang ohne Kühlung gerührt. Der ausgeschiedene Dicyclohexylharnstoff wird abfiltriert, dreimal mit 50 ml Tetrahydrofuran gewaschen, und die vereinigten Tetrahydrofuran-Lösungen werden im Vakuum eingedampft. Der Rückstand wird aus heissem Äthanol umkristallisiert, filtriert, zweimal mit 50 ml Äthanol bei 5 - 10°C gewaschen und im Vakuumexsiccator getrocknet.
Ausbeute: 72 g (75 %)
Schmelzpunkt: 139 - 141°C
$[\alpha]_D^{20}$ = +37° (c = 1, Dimethylformamid)

Analyse für $C_{23}H_{18}O_4NCl_3$ (478,75)

| | | | | | | | | | |
|---|---|---|---|---|---|---|---|---|---|
| berechnet: | C | 57,70 | H | 3,79 | N | 2,92 | Cl | 22,22 | % |
| gefunden: | C | 57,7 | H | 3,9 | N | 3,0 | Cl | 22,2 | % |

Stufe B: Benzyloxycarbonyl-D-phenylalanyl-L-prolin

71,8 g (0,15 Mol) Benzyloxycarbonyl-D-phenylalanin-2,4,5-trichlorphenylester (Beispiel 1, Stufe A) werden unter Rühren bei Raumtemperatur in 180 ml wasserfreiem Pyridin gelöst, dann wird die Lösung mit 17,3 g (0,15 Mol) L-Prolin und 21,0 ml (0,15 Mol) Triäthylamin versetzt. Das Rühren wird bis zur vollständigen Lösung des Prolins fortgesetzt, dann wird das Reaktionsgemisch 4 - 6 Stunden lang stehen gelassen, nachfolgend auf 80 - 100 ml auf dem Wasserbad bei max. 40°Cund 13 - 20 Millibar eingedampft und der Rückstand mit 250 ml Wasser und 90 ml Diäthyläther versetzt. Die wässrige Phase wird zweimal mit 30 ml Diäthyläther extrahiert, die vereinigten ätherischen Phasen zweimal mit 30 ml 5 %-iger

Natriumbicarbonat-Lösung gewaschen, dann werden die vereinigten wässrigen Phasen mit 3 n Salzsäure auf pH 2 angesäuert. Das ausgeschiedene Öl wird dreimal mit je 120 ml Äthylacetat extrahiert, die vereinigten Äthylacetat-Lösungen werden dreimal mit je 40 ml Wasser gewaschen, über wasserfreiem Natrium-sulfat getrocknet und am Wasserbad bei max. 40°C und 15 - 20 Millibar auf 50 - 100 ml eingedampft. Der Rückstand wird aus 200 ml Äther umkristallisiert. Die Kristalle werden abfiltriert, zweimal mit 40 ml Äther gewaschen und an der Luft getrocknet.
Ausbeute: 50,4 g (85 %)
Schmelzpunkt: 132 - 133°C
$[\alpha]_D^{20}$ = -46,4° (c=2, Dimethylformamid)
$R_F^3$ = 0,5

| Analyse für $C_{22}H_{24}O_5N_2$ (396,43) | | | | |
|---|---|---|---|---|
| berechnet: | C 66,65 | H 6,10 | N 7,07 | % |
| gefunden: | C 66,4 | H 6,25 | N 7,2 | %. |

Stufe C: N-Benzyloxycarbonyl-N-methyl-D-phenylalanyl-L-prolin-Cyclohexylammoniumsalz

39,6 g (0,1 Mol) Benzyloxycarbonyl-D-phenylalanyl-L-prolin (Beispiel 1, Stufe B) und 50 ml (0,8 Mol) Methyljodid werden in 300 ml wasserfreiem Tetrahydrofuran gelöst. Die Lösung wird auf 0°C abgekühlt und unter Rühren in 8-10 Portionen mit 13,2 g (0,3 Mol) Natriumhydrid versetzt, dann wird das Rühren bei Raumtemperatur weitere 2 Stunden lang fortgesetzt. Nachfolgend werden 500 ml Äthylacetat zu dem Reaktionsgemisch zwecks Zersetzung des gebildeten Natriumhydroxids gegeben und zusätzlich Vorsichtig noch 10 ml Wasser für die Zersetzung des überflüssigen Natriumhydrids. Die Lösung wird dann am Wasserbad bei max. 40°C und 15-20 Millibar eingedampft. Der Rückstand wird in 250 ml Wasser und 100 ml Diäthyläther gelöst, die wässrige Phase wird mit 100 ml Diäthyläther und die vereinigten Diäthyläther Phasen mit 50 ml 5 %-igem Natriumbicarbonat gewaschen. Die Natriumbicarbonat-Lösung und die wässrige Phase werden vereinigt, mit 5 n Kaliumbisulfat auf pH 2 angesäuert und zweimal mit 150 ml Äthylacetat extrahiert. Die vereinigten Äthylacetat-Phasen werden mit 100 ml Wasser, 50 ml 10 %-iger Natrium-thiosulfat-Lösung, wiederholt mit 100 ml Wasser gewaschen, über wasserfreiem Natriumsulfat getrocknet und am Wasserbad bei max. 40°C und 15 - 20 Millibar eingedampft. Der Rückstand wird in 600 ml Benzol gelöst, mit 12 ml (0,105 Mol) Cyclohexylamin versetzt und über Nacht bei Raumtemperatur stehen gelassen. Die ausgeschiedenen Kristalle werden abfiltriert, zweimal mit 200 ml Benzol gewaschen und im Vakuumexsiccator über Paraffinspänen getrocknet.
Ausbeute: 38,2 g (75 %)
$R_F^3$ = 0,53 - 0,60 (und Cyclohexylamin: 0,01 - 0,05)
Schmelzpunkt: 160 - 163°C
$[\alpha]_D^{20}$ = + 12,1° (c=1, Methanol)

| Analyse für $C_{23}H_{26}O_5N_2 \cdot C_6H_{13}N$ (509,63) | | | | |
|---|---|---|---|---|
| berechnet: | C 68,34 | H 7,71 | N 8,25 | % |
| gefunden: | C 68,5 | H 7,8 | N 8,3 | %. |

<u>$N^\alpha$-t-Butyloxycarbonyl-$N^\omega$-benzyloxycarbonyl-L-arginin-lactam</u>

Stufe D.: $N^\alpha$-t-Butyloxycarbonyl-$N^\omega$-benzyloxycarbonyl-L-arginin-hydrat

65,8 (0,2 Mol) t-Butyloxycarbonyl-L-arginin-chlorhydrat-hydrat [D. Yamashiro und Mitarbeiter: J. Am. Chem. Soc. 94, 2855-2859 (1972)] werden in 100 ml 4 n Natriumhydroxid gelöst, die Lösung wird auf 5 -

10°C gekühlt, dann unter Rühren mit 80 ml (0,5 Mol) Benzyloxycarbonylchlorid und ungefähr 150 ml 4 n Natriumhydroxid versetzt, so daß der pH-Wert der Lösung über 12 sei. Nachfolgend wird das Reaktionsgemisch noch 3 - 4 Stunden lang gerührt, dann wird es mit 150 ml Wasser verdünnt und mit 100 ml Diäthyläther extrahiert. Drei Phasen werden gebildet. Die unteren zwei Phasen werden noch zweimal mit 100 ml Diäthyläther gewaschen,mit 300 - 400 ml Methanol vermischt, um die Phasen zu homogenisieren, und der pH-Wert der Lösung wird, falls nötig, auf 12 eingestellt (4 n Natriumhydroxid). Nach 6 - 8-stündigem Stehen wird die Lösung zweimal mit einem 1:1 Gemisch von Diäthyläther-Petroläther extrahiert, der pH-Wert mit Eisessig auf 6 - 7 eingestellt (ungefähr 50 ml), und dreimal mit 140 ml Methylenchlorid extrahiert. Die Methylenchlorid-Lösungen werden vereinigt, zweimal mit 60 ml Wasser gewaschen und am Wasserbad bei 40°C und 25 - 30 Millibar eingedampft. Der Rückstand wird mit 300 ml Benzol und 600 ml Wasser versetzt, 2 - 3 Stunden lang bei 20°C gerührt, dann über Nacht bei 10°C stehen Die ausgeschiedenen Kristalle werden abfiltriert, mit 300 ml Wasser und 300 ml Benzol gewaschen, und im Vakuumexsiccator über Phosphorpentoxid und Paraffinspänen getrocknet.
Ausbeute: 60 g (70 %)
Schmelzpunkt: 122 - 124°C
$[\alpha]_D^{20}$ = -20,7° (c = 1, Pyridin)

| Analyse für $C_{19}H_{28}O_6N_4 \cdot H_2O$ (426,46) | | | | | | | |
|---|---|---|---|---|---|---|---|
| berechnet: | C | 53,51 | H | 7,09 | N | 13,14 | % |
| gefunden: | C | 53,35 | H | 7,2 | N | 13,1 | %. |

Stufe E: $N^\alpha$-t-Butyloxycarbonyl-$N^\omega$-benzyloxycarbonyl-L-arginin-lactam

59,7 g (0,14 Mol) $N^\alpha$-t-Butyloxycarbonyl-$N^\omega$-benzyloxycarbonyl-L-arginin-hydrat(Beispiel 1, Stufe D) und 19,6 g (0,14 Mol) Triäthylamin werden in 200 ml Tetrahydrofuran gelöst. Die Lösung wird auf -10°C gekühlt, und bei dieser Temperatur unter Rühren erst mit 18,5 ml (0,14 Mol) Isobutyloxycarbonylchlorid, dann nach 10 Minuten mit 19,6 ml (0,14 Mol) Triäthylamin versetzt, eine weitere Stunde lang bei 0°C und noch eine Stunde lang ohne Kühlung gerührt, und am Ende unter Rühren auf ein Liter Eiswasser geschüttet. Die ausgeschiedenen Kristalle werden abfiltriert, zweimal mit 100 ml Eiswasser gewaschen und nachfolgend in 280 ml Chloroform gelöst. Die Chloroform-Lösung wird mit 100 ml Eiswasser gewaschen, über wasserfreiem Calciumchlorid getrocknet und bei max. 40°C und 20 - 25 Millibar am Wasserbad eingedampft. Der kristalline Rüokstand wird in 70 ml Petroläther suspendiert, filtriert, zweimal mit 30 ml Petroläther gewaschen und im Vakuum exsiccator über Phosphorpentoxid getrocknet.
Ausbeute: 44 g (81 %)
Schmelzpunkt: 164 - 166°C
$[\alpha]_D^{20}$ = -24° (c = 1, Tetrahydrofuran)

| Analyse für $C_{19}H_{26}O_5N_4$ (390,43) | | | | | | | |
|---|---|---|---|---|---|---|---|
| berechnet: | C | 58,44 | H | 6,71 | N | 14,35 | % |
| gefunden: | C | 58,7 | H | 6,8 | N | 14.1 | %. |

## Beispiel 2

### N,N-Dimethyl-D-phenylalanyl-L-prolyl-L-argininaldehyd-sulfat

Stufe 1: N,N-Dimethyl-D-phenylalanyl-L-prolyl-$N^\omega$-benzyloxycarbonyl-L-arginin-lactam-hydrochlorid

4,3 g (0,011 Mol) $N^\alpha$-t-Butyloxycarbonyl-$N^\omega$-benzyloxycarbonyl-L-arginin-lactam(Beispiel 1, Stufe E) werden gemäss dem Verfahren von Beispiel 1, Stufe 1 unter Anwendung von proportionalen Mengen von Reagenzien und Lösungen umgesetzt. Die erhaltene Suspension wird zu dem folgenden gemischten

Anhydrid gegeben:

3,26 g (0,01 Mol) N,N-Dimethyl-D-phenylalanyl-L-prolin-hydrat und 2,2 ml (0,02 Mol) N-Methylmorpholin werden in einem Gemisch von 50 ml Benzol und 5 ml Äthanol gelöst. Die Lösung wird am Wasserbad bei 40°C und 40 - 50 Millibar auf 10 - 15 ml eingeengt, dann werden noch viermal je 40 ml Benzol von dem Konzentrat abdestilliert, beim letzten Abdestillieren wird zur Trockne eingedampft. Der Rückstand wird in 10 ml Dimethylformamid gelöst und auf -15°C gekühlt. Bei dieser Temperatur wird er mit 1,32 ml (0,01 Mol) Isobutyloxycarbonylchlorid und nach fünf Minuten mit der obigen Dimethylformamid-Suspension versetzt. Das Reaktionsgemisch wird eine Stunde lang bei -15°C und eine weitere Stunde lang bei 0°C gerührt, dann wird es mit 40 ml Äthylacetat versetzt und die ausgeschiedenen Kristalle werden abfiltriert. Dem Filtrat werden 20 ml Wasser zugefügt und die Phasen werden abgeschieden. Die wässrige Dimethylformamid-Phase wird dreimal mit je 5 ml Äthylacetat extrahiert, die vereinigten Äthylacetat Phasen werden zweimal mit 15 ml Wasser, zweimal mit 15 ml 5 %-iger Natriumbicarbonat-Lösung und wieder zweimal mit 15 ml Wasser gewaschen, über wasserfreiem Natriumsulfat getrocknet und am Wasserbad bei 40°C und 20 - 25 Millibar auf 10 - 15 ml eingeengt. Das Konzentrat wird mit so viel salzsauerem Äthylacetat versetzt, dass die Säure in der Gasphase nachweisbar sei, dann wird es mit 30-40 ml Diäthyläther verdünnt. Der ausgeschiedene Niederschlag wird abfiltriert, mit Diäthyläther gewaschen und im Vakuumexsiccator über Schwefelsäure und Kaliumhydroxid getrocknet.

Ausbeute: 2,7 g (45 %)
$R_F^4$ = 0,43
$[\alpha]_D^{20}$ = - 56,0° (c = 1, Chloroform)

Analyse für $C_{30}H_{38}O_5N_6 \cdot 9/5\ HCl \cdot 2H_2O$ (666,13)

| | | | | | | | | | | |
|---|---|---|---|---|---|---|---|---|---|---|
| berechnet: | C | 54,09 | H | 6,90 | N | 12,62 | Cl | 9,58 | % | |
| gefunden: | C | 54,0 | H | 7,0 | N | 12,6 | Cl | 9,8 | % | . |

Stufe 2: N,N-Dimethyl-D-phenylalanyl-L-prolyl-$N^\omega$-benzyloxycarbonyl-L-argininaldehyd-sulfat

2,4 g (0,004 Mol) N,N-Dimethyl-D-phenylalanyl-L-prolyl-$N^\omega$-benzyloxycarbonyl-L-arginin-lactam-hydrochlorid (Beispiel 2, Stufe 1) werden in 15 ml Tetrahydrofuran gelöst, die Lösung wird auf -20°C abgekühlt und unter Rühren 0,003 Mol Lithium-aluminium-hydrid in Tetrahydrofuran-Lösung zugefügt. Der Fortschritt der Reduktion wird mit Dünnschichtchromatographie im Lösungsmittelsystem Äthylacetat-Pyridin-Essigsäure-Wasser (60:20:6:11) verfolgt ($R_F$-Werte des Lactams und des Aldehyds o,35 bzw. 0,2). Wenn nötig, werden weitere Portionen der Lithium-aluminium-hydrid-Lösung zugefügt, dann wird das Reaktionsgemisch unter Kühlung und Rühren mit 1 n Schwefelsäure auf pH 5 angesäuert. Die Lösung wird bis zur Opaleszenz mit Wasser verdünnt (ungefähr 30 ml) und nachfolgend zweimal mit 15 ml Chloroform extrahiert. Dann wird die wässrige Tetrahydrofuran-Phase dreimal mit 20 ml n-Butanol extrahiert, die vereinigten n-Butanol-Phasen mit mit n-Butanol gesättigtem Wasser (10 ml) gewaschen und am Wasserbad bei max. 40°C und 15 - 20 Millibar auf 5 - 10 ml eingeengt. Der Rückstand wird mit 30 ml Äther verdünnt, der ausgeschiedene Niederschlag wird abfiltriert, zweimal mit 10 ml Diäthyläther gewaschen, dann wird er in einem Gemisch von 20 ml Chloroform und 5 ml Methanol gelöst. Der unlösliche Teil wird abfiltriert und das Filtrat wie oben aufgearbeitet.

Der ölige Rückstand wird mit 30 ml Diäthyläther verarbeitet, der Niederschlag abfiltriert, zweimal mit 20 ml Äther und zweimal mit 20 ml Petroläther gewaschen und im Vakuumexsiccator über Paraffinstäbchen, Schwefelsäure und Kaliumhydroxid getrocknet.

Ausbeute: 2,25 g (85 %)
$R_F^5$ = 0,48 - 0,54

Analyse für $C_{30}H_{38}O_5N_6.H_2SO_4$ (660,73)

| | | | | | | | | |
|---|---|---|---|---|---|---|---|---|
| berechnet: | C | 54,53 | H | 6,10 | N | 12,72 | S | 4,84 % |
| gefunden: | C | 55,0 | H | 6,5 | N | 12,1 | S | 4,7 % . |

Stufe 3: N,N-Dimethyl-D-phenylalanyl-L-prolyl-L-argininaldehyd-sulfat

1,32 g (0,002 Mol) N,N-Dimethyl-D-phenylalanyl-L-prolyl-$N^{\omega}$-benzyloxycarbonyl-L-argininaldehyd-sulfat (Beispiel 2, Stufe 2) werden in einem Gemisch von 5 ml entionisiertem Wasser und 15 ml Äthanol gelöst, und in Gegenwart von 0,2 g 10 %-igem Pd/C-Katalysator hydriert. Der Katalysator wird abfiltriert und zweimal mit 10 ml entionisiertem Wasser gewaschen. Das Filtrat und die Waschlösungen werden am Wasserbad bei max. 40°C und 15 - 20 Millibar auf 5 - 10 ml eingeengt, dann mit entionisertem Wasser auf 20 ml verdünnt und der pH-Wert, wenn nötig, entweder mit 0,1 n Schwefelsäure oder mit einem Ionenaustauscher in $OH^-$-Cyclus (z.B. AG 1X8) auf 6,0-6,5 eingestellt. Nachfolgend wird die wässrige Lösung lyophilisiert.
Ausbeute: 0,85 g (80 %)
$R_F^6$ = 0,35 - 0,39

Analyse für $C_{22}H_{34}O_3N_6.H_2SO_4.3(H_2O)$ (566,67)

| | | | | | | | | |
|---|---|---|---|---|---|---|---|---|
| berechnet: | C | 46,60 | H | 7,47 | N | 14,82 | S | 5,66 % |
| gefunden: | C | 45,5 | H | 7,4 | N | 14,3 | S | 5,2 % . |

Die Ausgangssubstanz N,N-Dimethyl-D-phenylalanyl-L-prolin-hydrat kann wie folgt hergestellt werden:

11,9 g (0,03 Mol) Benzyloxycarbonyl-D-phenylalanyl-L-prolin (Beispiel 1, Stufe B) werden in 200 ml 50 %-igem wässrigem Methanol gelöst, mit 9,7 ml (ungefähr 0,12 Mol) 37 %-iger wässriger Formaldehyd-Lösung und 6 g 10 %-igem Pd/C-Katalysator versetzt, und hydriert. Der Fortschritt der Reaktion wird dünnschichtchromatographisch im Lösungsmittelsystem Äthylacetat-Pyridin-Essigsäure-Wasser (30:20:6:11) verfolgt ($R_F$-Werte des Ausgangsstoffes bzw. Endproduktes: 0,95, bzw. 0,45). Nach beendigter Reaktion wird der Katalysator abfiltriert, zweimal mit 50 ml wässrigem Methanol gewaschen und die vereinigten Lösungen werden am Wasserbad bei 40°C und 20 - 25 Millibar eingedampft. Der Rückstand wird in 60 ml Äthylacetat gelöst, mit 0,5 ml Wasser versetzt und über Nacht bei 5 - 10°C stehen gelassen. Die ausgeschiedenen Kristalle werden abfiltriert, zweimal mit 15 ml Äthylacetat, das auf 5 - 10°Cabgekühlt ist, gewaschen und im Vakuumexsiccator über Paraffinspänen, Schwefelsäure und Kaliumhydroxid getrocknet.
Ausbeute: 6,5 g (67 %)
Schmelzpunkt: 228 - 229°C
$[\alpha]_D^{20}$ = -159° (c = 1, Wasser)

Analyse für $C_{16}H_{22}O_3N_2.H_2O$ (308,37)

| | | | | | | |
|---|---|---|---|---|---|---|
| berechnet: | C | 62,31 | H | 7,85 | N | 9,08 % |
| gefunden: | C | 62,5 | H | 8,0 | N | 8,95 %. |

Beispiel 3

N-Äthyl-D-phenylalanyl-L-prolyl-L-argininaldehyd-sulfat

Stufe 1: N-Benzyloxycarbonyl-N-äthyl-D-phenylalanyl-L-prolyl-$N^{\omega}$-benzyloxycarbonyl-L-arginin-lactam

2,1 g (0,005 Mol) N-Benzyloxycarbonyl-N-äthyl-D-phenylalanyl-L-prolin und 2,15 g (0,0055 Mol) $N^{\alpha}$-t-Butyloxycarbonyl-$N^{\omega}$-benzyloxycarbonyl-L-arginin-lactam (Beispiel 1, Stufe E) werden gemäss dem Verfahren von Beispiel 1, Stufe 1 bei Anwendung von proportionalen Mengen von Reagenzien und Lösungsmitteln umgesetzt bzw. kondensiert. Bei der Aufarbeitung des Reaktionsgemisches und während der Säulenchromatographie werden die Fraktionen mit Dünnschichtchromatographie im System Benzol-Tetrahydrofuran (8:2) untersucht. Die Fraktionen, die das reine Hauptprodukt enthalten ($R_F$ = 0,4 - 0,5 ), werden vereinigt, am Wasserbad bei ungefähr 40°C und 15 - 20 Millibar eingedampft, der Rückstand wird mit Diisopropyläther verarbeitet, filtriert, mit Diisopropyläther gewaschen und im Vakuumexsiccator über Schwefelsäure und Paraffinspänen getrocknet.
Ausbeute: 1,9 g (54 %)
$R_F^2$ = 0,75 - 0,85.

Stufe 2: N-Benzyloxycarbonyl-N-äthyl-D-phenylalanyl-L-prolyl-$N^{\omega}$-benzyloxycarbonyl-L-argininaldehyd

1,4 g (0,002 Mol) N-Benzyloxycarbonyl-N-äthyl-D-phenylalanyl-L-prolyl-$N^{\omega}$-benzyloxycarbonyl-L-arginin-lactam(Beispiel 3, Stufe 1) werden mit dem Verfahren von Beispiel 1, Stufe 2 bei Anwendung von proportionalen Mengen von Reagenzien umgesetzt.
Ausbeute: 1,15 g (72 %) eines Produktes, das gemäss der Elementaranalyse 1 Mol Cyclohexan enthielt.
$R_F^3$ = 0,55 - 0,65

| | | | | | | | |
|---|---|---|---|---|---|---|---|
| Analyse für $C_{38}H_{46}O_7N_6 \cdot C_6H_{12}$ (782,95) | | | | | | | |
| berechnet: | C | 67,49 | H | 7,47 | N | 10,73 | % |
| gefunden: | C | 67,6 | H | 7,5 | N | 10,5 | % . |

Stufe 3: N-Äthyl-D-phenylalanyl-L-prolyl-L-argininaldehyd-sulfat

0,78 g (0,001 Mol) N-Benzyloxycarbonyl-N-äthyl-D-phenylalanyl-L-prolyl-$N^{\omega}$-benzyloxycarbonyl-L-argininaldehyd (Beispiel 3, Stufe 2) werden gemäss dem Verfahren von Beispiel 1, Stufe 3 bei Anwendung von proportionalen Mengen von Reagenzien und Lösungsmitteln umgesetzt.
Ausbeute: 0,75 g (70 %)
$R_F^6$ = 0,4 - 0,5

| | | | | | | | | | |
|---|---|---|---|---|---|---|---|---|---|
| Analyse für $C_{22}H_{34}O_3N_6 \cdot 5/6(H_2SO_4) \cdot 3,5(H_2O)$ (575,27) | | | | | | | | | |
| berechnet: | C | 45,93 | H | 7,48 | N | 14,61 | S | 4,64 | % |
| gefunden: | C | 46,1 | H | 7,6 | N | 14,2 | S | 4,6 | % . |

Die Ausgangssubstanz N-Benzyloxycarbonyl-N-äthyl-D-phenylalanyl-L-prolin kann folgendermassen hergestellt werden:

Stufe A: N-Äthyl-D-phenylalanin

8,25 g (0,05 Mol) D-Phenylalanin werden in 100 ml 20 %-igem wässrigem Äthanol gelöst, mit 5,65 ml (0,1 Mol) Acetaldehyd und 2 g 10 %-igem Pd/C-Katalysator versetzt und zwei Tage lang hydriert. Der Katalysator wird abfiltriert, mit 60 ml 2 n Salzsäure gewaschen und der pH-Wert der wässrigen Lösungen wird mit 4 n Natriumhydroxid auf 7 eingestellt. Das ausgeschiedene Produkt wird abfiltriert, dreimal mit 20 ml Wasser gewaschen und im Vakuumexsiccator über wasserfreiem Calciumchlorid getrocknet.
Ausbeute: 4,85 g (50 %)
$[\alpha]_D^{20}$ = -53° (c = 1, 0,1 n Natriumhydroxid)

Analyse für $C_{11}H_{15}O_2N$ (193,24)
berechnet: C 68,37 H 7,82 N 7,25 %
gefunden: C 68,5 H 7,65 N 7,2 %.

Stufe B: N-Benzyloxycarbonyl-N-äthyl-D-phenylalanin

2,9 g (0,015 Mol) N-Äthyl-D-phenylalanin (Beispiel 3, Stufe A) werden in 22,5 ml 2 n Natriumhydroxid gelöst, erst mit 10 ml Dioxan, dann nach Abkühlen auf 5 - 10°C mit 2,5 ml (ungefähr 0,017 Mol) Benzyloxycarbonylchlorid versetzt und unter Kühlung 3 Stunden lang gerührt. Das Reaktionsgemisch wird mit 40 ml Wasser verdünnt und mit einem 1:1 Gemisch von 30 ml Diäthyläther-Petroläther extrahiert. Ein Dreiphasensystem wird gebildet. Die unteren zwei Phasen werden wieder mit 30 ml Diäthyläther extrahiert und die vereinigten wässrigen Phasen mit 1 n Schwefelsäure angesäuert (pH = 2). Das ausgeschiedene Produkt wird dreimal mit 30 ml Äthylacetat extrahiert. Die Äthylacetat-Lösungen werden vereinigt, zweimal mit 20 ml Wasser gewaschen, über wasserfreiem Natriumsulfat getrocknet und dann am Wasserbad bei max. 40°C und 15 - 20 Millibar eingedampft.
Ausbeute: 2,7 g (55 %) öliges Produkt
$R_F^2$ = 0,5 - 0,6.

Stufe C: N-Benzyloxycarbonyl-N-äthyl-D-phenylalanyl-L-prolin

2,6 g (0,008 Mol) N-Benzyloxycarbonyl-N-äthyl-D-phenylalanin (Beispiel 3, Stufe B) werden in 10 ml Tetrahydrofuran gelöst, bei 0°C mit 1,6 g (0,008 Mol) 2,4,5-Trichlorphenol und 1,65 g (0,008 Mol) Dicyclohexylcarbodiimid versetzt und bei Raumtemperatur vier Stunden lang stehen gelassen. Das Reaktionsgemisch wird filtriert und am Wasserbad bei max. 40°C und 15 - 20 Millibar eingedampft. Der Rückstand wird in 40 ml Benzol gelöst, zweimal mit 20 ml 5 %-iger Natriumbicarbonat-Lösung und zweimal mit 20 ml Wasser gewaschen, nachfolgend über wasserfreiem Natriumsulfat getrocknet und am Wasserbad bei max.40°C und 15 - 20 Millibar eingedampft. Der ölige Rückstand wird in 10 ml Pyridin gelöst, erst mit 0,92 g (0,008 Mol) L-Prolin, dann mit 1,12 ml (0,008 Mol) Triäthylamin versetzt und bei Raumtemperatur bis zur vollständigen Lösung des Prolins gerührt. Die Lösung wird 4 - 5 Stunden lang stehen gelassen und nachfolgend am Wasserbad bei max. 40°C und 15 - 20 Millibar eingedampft. Der Rückstand wird in 25 ml Wasser und 10 ml Diäthyläther gelöst, die wässrige Phase wird zweimal mit 10 ml Diäthyläther und die vereinigten Diäthyläther-Phasen zweimal mit 10 ml 5 %-iger Natriumbicarbonat-Lösung gewaschen. Die vereinigten wässrigen Phasen werden mit 3 n Salzsäure auf pH = 2 angesäuert. Das ausgeschiedene Produkt wird dreimal mit 15 ml Benzol extrahiert, die vereinigten Benzolextrakte werden dreimal mit 5 ml Wasser gewaschen, über wasserfreiem Natriumsulfat getrocknet und am Wasserbad bei ungefähr 40°C und 15 - 20 Millibar eingedampft.
Ausbeute 2,2 g (64 %) öliges Produkt.
$R_F^5$ = 0,5 - 0,6.

## Beispiel 4

N-Isobutyl-D-phenylalanyl-L-prolyl-L-argininaldehyd-sulfat

Stufe 1: N-Benzyloxycarbonyl-N-isobutyl-D-phenylalanyl-L-prolyl-$N^{\omega}$-benzyloxycarbonyl-L-arginin-lactam

2,26 g (0,005 Mol) N-Benzyloxycarbonyl-N-isobutyl-D-phenylalanyl-L-prolin und $N^{\alpha}$-tert.-Butyloxycarbonyl-$N^{\omega}$-benzyloxycarbonyl-L-arginin-lactam (Beispiel 1, Stufe E) werden gemäß dem Verfahren von Beispiel 1, Stufe 1 bei Anwendung von proportionalen Mengen von Reagenzien und Lösungsmitteln umgesetzt bzw. kondensiert. Bei der Aufarbeitung des Reaktionsgemisches und während der Säulenchromatographie werden die Fraktionen dünnschichtchromatographisch im Lösungsmittelsystem Äthylacetat-Pyridin-Essigsäure-Wasser (480:20:6:11) geprüft, und die Fraktionen, die das reine Produkt mit $R_F^2$ = 0,75 - 0,85 enthalten, vereinigt. Sie werden am Wasserbad bei ungefähr 40°C und 15 - 20 Millibar eingedampft, der Rückstand wird mit Petroläther verarbeitet, filtriert, mit Petroläther gewaschen und im Vakuumexsiccator

über Paraffinspänen getrocknet.
Ausbeute: 2,7 g (75 %)
$R_F^2$ = 0,75 - 0,85
$[\alpha]_D^{20}$ = -5,8° (c = 1, Tetrahydrofuran).

| | | | | |
|---|---|---|---|---|
| Analyse auf $C_{40}H_{48}O_7N_6 \cdot 2/3(C_4H_8O)$ (772,90) | | | | |
| berechnet: | C 66,30 | H 6,95 | N 10,87 | |
| gefunden: | C 65,0 | H 6,8 | N 10,80 | . |

Stufe 2: N-Benzyloxycarbonyl-N-isobutyl-D-phenylalanyl-L-prolyl-N$^\omega$-benzyloxycarbonyl-L-argininaldehyd

1,45 g (0,002 Mol) N-Benzyloxycarbonyl-N-isobutyl-D-phenylalanyl-L-prolyl-N$^\omega$-benzyloxycarbonyl-L-arginin-lactam (Beispiel 4, Stufe 1) werden gemäss dem Verfahren von Beispiel 1, Stufe 2 bei Anwendung von proportionalen Mengen von Reagenzien und Lösungsmitteln reduziert.
Ausbeute: 1,0 g (62 %)
$R_F^3$ = 0,24
$[\alpha]_D^{20}$ = +2,3 (c = 1, Tetrahydrofuran)

| | | | | |
|---|---|---|---|---|
| Analyse auf $C_{40}H_{50}O_7N_6 \cdot 1/4(C_6H_{12})$ (747,89) | | | | |
| berechnet: | C 66,64 | H 7,14 | N 11,24 % | |
| gefunden: | C 66,3 | H 7,1 | N 11,2 % | . |

Stufe 3: N-Isobutyl-D-phenylalanyl-L-prolyl-L-argininaldehyd-sulfat

0,81 g (0,001 Mol) N-Benzyloxycarbonyl-N-isobutyl-D-phenylalanyl-L-prolyl-N$^\omega$-benzyloxycarbonyl-L-argininaldehyd (Beispiel 4, Stufe 2) werden gemäss dem Verfahren von Beispiel 1, Stufe 3 bei Anwendung von proportionalen Mengen von Reagenzien und Lösungsmitteln umgesetzt.
Ausbeute: 0,45 g (80 %)
$R_F^6$ = 0,64

| | | | | | |
|---|---|---|---|---|---|
| Analyse für $C_{24}H_{38}O_3N_6 \cdot H_2SO_4 \cdot 4(H_2O)$ (628,74) | | | | | |
| berechnet: | C 45,84 | H 7,69 | N 13,36 | S 5,1 % | |
| gefunden: | C 45,8 | H 7,2 | N 13,4 | S 4,9 % | . |

Die Ausgangssubstanz N-Benzyloxycarbonyl-N-isobutyl-D-phenylalanyl-L-prolin wird gemäss folgendem Verfahren hergestellt:

Stufe A: N-Isobutyl-D-phenylalanin

Aus 8,25 g (0,05 Mol) D-Phenylalanin ausgehend wird gemäss dem Verfahren von Beispiel 3, Stufe A gearbeitet mit dem Unterschied, dass 9,1 ml (0,1 Mol) Isobutyraldehyd statt Acetaldehyd verwendet werden.
Ausbeute: 8,7 g (70 %)
$[\alpha]_D^{20}$ = -29,9° (c = 1, 0,1 n Natriumhydroxid)

Analyse für $C_{13}H_{19}O_2N.1/2(H_2O)$ (230,30)

| | | | |
|---|---|---|---|
| berechnet: | C 67,79 | H 8,75 | N 6,08 % |
| gefunden: | C 67,2 | H 8,9 | N 6,15 % . |

Stufe B: N-Benzyloxycarbonyl-N-isobutyl-D-phenylalanin

3,32 g (0,015 Mol) N-Isobutyl-D-phenylalanin (Beispiel 4, Stufe A) werden gemäss dem Verfahren von Beispiel 3, Stufe B umgesetzt.
Ausbeute: 3,2 g (60 %) öliges Produkt
$R_F^2$ = 0,6 - 0,7.

Stufe C: N-Benzyloxycarbonyl-N-isobutyl-D-phenylalanyl-L-prolin

2,85 g (0,008 Mol) N-Benzyloxycarbonyl-N-isobutyl-D-phenylalanin (Beispiel 4, Stufe B) werden gemäß dem Verfahren von Beispiel 3, Stufe C umgesetzt.
Ausbeute: 2,45 g (67 %) öliges Produkt
$R_F^5$ = 0,65 - 0,70.

Beispiel 5

N-Methyl-D-alloisoleucyl-L-prolyl-L-argininaldehyd-sulfat

Stufe 1: N-Benzyloxycarbonyl-N-methyl-D-alloisoleucyl-L-prolyl-$N^{\omega}$-benzyloxycarbonyl-L-arginin-lactam

4,3 g (0,011 Mol) $N^{\alpha}$-t-Butyloxycarbonyl-$N^{\omega}$-benzyloxycarbonyl-L-arginin-lactam(Beispiel 1, Stufe E) und 4,8 g (0,01 Mol) N-Benzyloxycarbonyl-N-methyl-D-alloisoleucyl-L-prolin-Cyclohexylammoniumsalz werden gemäss dem Verfahren von Beispiel 1, Stufe 1 bei Anwendung von proportionalen Mengen von Reagenzien und Lösungsmitteln umgesetzt bzw. kondensiert. Während der Säulenchromatographie werden die Fraktionen, die das Produkt mit $R_F^2$ = 0,74 - 0,80 rein enthalten, vereinigt, eingedampft, der Rückstand wird mit 40 ml Diisopropyläther verarbeitet, filtriert, zweimal mit 20 ml Diisopropyläther gewaschen und im Vakuumexsiccator über Schwefelsäure und Paraffinstäbchen getrocknet.
Ausbeute: 4,3 g (66 %)
$R_F^2$ = 0,74 - 0,80

Analyse für $C_{34}H_{44}O_7N_6$ (648,74)

| | | | |
|---|---|---|---|
| berechnet: | C 62,94 | H 6,84 | N 12,96 % |
| gefunden: | C 63,1 | H 6,9 | N 12,7 %. |

Stufe 2: N-Benzyloxycarbonyl-N-methyl-D-alloisoleucyl-L-prolyl-$N^{\omega}$-benzyloxycarbonyl-L-argininaldehyd

3,25 g (0,005 Mol) N-Benzyloxycarbonyl-N-methyl-D-alloisoleucyl-L-prolyl-$N^{\omega}$-benzyloxycarbonyl-L-arginin-lactam (Beispiel 5, Stufe 1) werden gemäss dem Verfahren von Beispiel 1, Stufe 2 bei Anwendung von proportionalen Mengen von Reagenzien und Lösungsmitteln umgesetzt.
Ausbeute: 2,5 g (66 %) eines Produktes, das gemäss der Elementaranalyse 1 Mol Cyclohexan enthält.
$R_F^3$ = 0,55

Analyse für $C_{34}H_{46}O_7N_6 \cdot C_6H_{12}$ (734,91)

| | | | | | | |
|---|---|---|---|---|---|---|
| berechnet: | C | 65,37 | H | 7,95 | N | 11,43 % |
| gefunden: | C | 65,0 | H | 7,8 | N | 11,6 % . |

Stufe 3: N-Methyl-D-alloisoleucyl-L-prolyl-L-argininaldehyd-sulfat

1,47 g (0,002 Mol) N-Benzyloxycarbonyl-N-methyl-D-alloisoleucyl-L-prolyl-$N^{\omega}$-benzyloxycarbonyl-L-argininaldehyd (Beispiel 5, Stufe 2) werden gemäss dem Verfahren von Beispiel 1, Stufe 3 bei Anwendung von proportionalen Mengen von Reagenzien und Lösungsmitteln umgesetzt.
Ausbeute: 0,85 g (87 %)
$R_F^6$ = 0,4

Analyse für $C_{18}H_{34}O_3N_6 \cdot H_2SO_4 \cdot 4(H_2O)$ (552,65)

| | | | | | | | | |
|---|---|---|---|---|---|---|---|---|
| berechnet: | C | 39,12 | H | 8,02 | N | 15,21 | S | 5,80 % |
| gefunden: | C | 39,2 | H | 7,70 | N | 15,1 | S | 5,7 % . |

Die Ausgangssubstanz , N-Benzyloxycarbonyl-N-methyl-D-alloisoleucyl-L-prolin-Cyclohexylammoniumsalz wird gemäss folgendem Verfahren hergestellt:

Stufe A: N-Benzyloxycarbonyl-D-alloisoleucyl-L-prolin-Cyclohexylammonium salz

13,3 g (0,05 Mol) Benzyloxycarbonyl-D-alloisoleucin [M . Winitz und Mitarbeiter: J. Am. Chem. Soc. 98, 2423-2430 (1956)] und 9,9 g (0,05 Mol) 2,4,5-Trichlorphenol werden in 50 ml Äthylacetat gelöst, die Lösung wird mit 10,2 g (0,05 Mol) Dicyclohexylcarbodiimid versetzt und über Nacht stehen gelassen. Der ausgeschiedene Dicyclohexylharnstoff wird abfiltriert, und das Filtrat am Wasserbad bei ungefähr 40°C und 15 - 20 Millibar eingedampft. Der Rückstand wird in 100 ml n-Hexan gelöst, erst mit 20 ml 1 n Natriumhydroxid, dann zweimal mit 20 ml Wasser extrahiert, nachfolgend wird die Lösung über Natriumsulfat getrocknet und wie oben eingedampft. Der Rückstand wird in 50 ml Pyridin gelöst, die Lösung wird mit 5,7 g (0,05 Mol) L-Prolin und 7,0 ml (0,05 Mol) Triäthylamin versetzt, bis zur vollständigen Lösung des Prolins gerührt und nachfolgend über Nacht stehen gelassen. Das Reaktionsgemisch wird am Wasserbad bei ungefähr 40°C und 15 - 20 Millibar eingedampft und der Rückstand in 50 ml 5 %-iger Natriumbicarbonat-Lösung und 50 ml Diäthyläther gelöst. Die wässrige Phase wird zweimal mit 30 ml Diäthyläther gewaschen, dann wird sie mit 3 n Salzsäure auf pH = 3 angesäuert. Das ausgeschiedene Produkt wird dreimal mit 30 ml Äthylacetat extrahiert, die vereinigten Äthylacetat-Lösungen werden dreimal mit 30 ml Wasser gewaschen, über wasserfreiem Natriumsulfat getrocknet und wie oben eingedampft. Der Rückstand wird in 100 ml Diisopropyläther gelöst, mit 6,0 ml (0,052 Mol) Cyclohexylamin versetzt und 4 - 5 Stunden lang stehen gelassen. Die ausgeschiedenen Kristalle werden abfiltriert, dreimal mit 30 ml Diisopropyläther gewaschen und im Vakuumexsiccator über Schwefelsäure und Kaliumhydroxid getrocknet.
Ausbeute: 14,5 g (62 %)
Schmelzpunkt: 138 - 140°C
$R_F^3$ = 0,35 - 0,40
$[\alpha]_D^{20}$ = -30,7° (c=1, Methanol)

Analyse für $C_{19}H_{26}O_5N_2 \cdot C_6H_{13}N$ (461,59)
berechnet: C 65,05 H 8,52 N 9,10 %
gefunden: C 65,4 H 8,5 N 9,05 % .

Stufe B: N-Benzyloxycarbonyl-N-methyl-D-alloisoleucyl-L-prolin-Cyclohexylammoniumsalz

9,25 g (0,02 Mol) Benzyloxycarbonyl-D-alloisoleucyl-L-prolin-Cyclohexylammoniumsalz (Beispiel 5, Stufe A) werden in 50 ml Diäthyläther und 50 ml 0,5 n Schwefelsäure gelöst. Die wässrige Phase wird mit 20 ml Diäthyläther extrahiert, die vereinigten ätherischen Phasen dreimal mit 30 ml Wasser gewaschen, über wasserfreiem Natriumsulfat getrocknet, dann am Wasserbad bei ungefähr 40°C und 20 - 30 Millibar eingedampft. Der Rückstand - N-Benzyloxycarbonyl-D-alloisoleucyl-L-prolin - wird gemäss dem Verfahren von Beispiel 1, Stufe C bei Anwendung von proportionalen Mengen von Reagenzien und Lösungsmitteln umgesetzt.
Ausbeute: 8,1 g (85 %)
Schmelzpunkt: 120 - 123°C
$R_F^3$ = 0,55 - 0,60
$[\alpha]_D^{20}$ = +43,8° (c=1, Methanol)

Analyse für $C_{20}H_{28}O_5N_2 \cdot C_6H_{13}N$ (475,61)
berechnet: C 65,65 H 8,69 N 8,83 %
gefunden: C 65,6 H 8,7 N 8,75 % .

Beispiel 6

N-n-Hexyl-D-phenylalanyl-L-prolyl-L-argininaldehyd-sulfat

Stufe 1: N-Benzyloxycarbonyl-N-n-hexyl-D-phenylalanyl-L-prolyl-$N^\omega$-benzyloxycarbonyl-L-argininaldehyd

3,5 g (0,01 Mol) N-n-Hexyl-D-phenylalanyl-L-prolin werden in einem Gemisch von 10 ml Dioxan und 14 ml 2 n Natriumhydroxid gelöst, die auf 5 - 10°C abgekühlte Lösung wird mit 2,1 ml (0,028 Mol) Benzyloxycarbonylchlorid versetzt und 4 Stunden lang gerührt. Nachfolgend wird das Reaktionsgemisch mit 30 ml Wasser verdünnt und zweimal mit 20 ml Petroläther extrahiert. Die wässrige Phase wird mit 1 n Schwefelsäure auf pH = 2-3 angesäuert und dreimal mit 20 ml Äthylacetat extrahiert. Die vereinigten Äthylacetat-Lösungen werden dreimal mit 10 ml Wasser gewaschen, über wasserfreiem Natriumsulfat getrocknet, dann am Wasserbad bei 20-25 Millibar eingedampft.
Ausbeute: 2,9 g (60 %) öliges Produkt
$R_F^2$ = 0,48.

Dieses Produkt und 2,58 g (0,0066 Mol) $N^\alpha$-t-Butyloxycarbonyl-$N^\omega$-benzyloxycarbonyl-L-arginin-lactam (Beispiel 1, Stufe E) werden gemäss dem Verfahren Von Beispiel 1, Stufe 1 bei Anwendung von proportionalen Mengen von Reagenzien und Lösungsmitteln umgesetzt bzw. kondensiert. Bei der Säulenchromatographie werden die Fraktionen, die das Hauptprodukt ($R_F^2$ = 0,68) rein enthalten, vereinigt und bei 20 - 25 Millibar eingedampft.
Ausbeute: 2,7 g (60 %)
$R_F^2$ = 0,68 .

Dieses Produkt wird gemäss dem Verfahren von Beispiel 1, Stufe 2 bei Anwendung von proportionalen Mengen von Reagenzien und Lösungsmitteln umgesetzt.
Ausbeute: 2,2 g (80 %)
$R_F^2$ = 0,53

Analyse für $C_{42}H_{54}O_7N_6$ (699,82)
berechnet: C 66,82 H 7,21 N 11,13 %
gefunden: C 66,5 H 7,4 N 11,4 % .

Stufe 2: N-n-Hexyl-D-phenylalanyl-L-prolyl-L-argininaldehyd-sulfat

0,74 g (0,001 Mol) N-Benzyloxycarbonyl-N-n-hexyl-D-phenylalanyl-L-prolyl-$N^{\omega}$-benzyloxycarbonyl-L-argininaldehyd (Beispiel 6, Stufe 1) werden gemäss dem Verfahren von Beispiel 1, Stufe 3 bei Anwendung von proportionalen Mengen von Reagenzien und Lösungsmitteln umgesetzt.
Ausbeute: 0,52 g (80 %)
$R_F^6$ = 0,75

Analyse für $C_{26}H_{42}O_3N_6 \cdot H_2SO_4 \cdot 4(H_2O)$ (656,79)
berechnet: C 47,54 H 7,98 N 12,80 S 4,88 %
gefunden: C 47,6 H 8,1 N 12,7 S 4,8 % .

Die Ausgangssubstanz N-n-Hexyl-D-phenylalanyl-L-prolin kann gemäss dem folgenden Verfahren hergestellt werden:

7,9 g (0,02 Mol) N-Benzyloxycarbonyl-D-phenylalanyl-L-prolin (Beispiel 1, Stufe B) und 4,9 g (0,04 Mol) Capronaldehyd werden in 100 ml 80 %-igem Äthanol gelöst und in der Gegenwart von 6 g 10 %-igem Pd/C-Katalysator hydriert. Nach beendeter Reaktion wird der Katalysator abfiltriert, das Filtrat eingedampft, der kristalline Rückstand wird in 20 ml Wasser suspendiert, filtriert, zweimal mit 10 ml Wasser gewaschen und im Vakuumexsiccator über Phosphorpentoxid getrocknet.
Ausbeute: 3,0 g (60 %)
$R_F^5$ = 0,6
$[\alpha]_D^{20}$ = -97,3° (c = 1, 0,1 n Natriumhydroxid)

Analyse für $C_{20}H_{30}O_3N_2$ (346,45)
berechnet: C 69,33 H 8,73 N 8,08 %
gefunden: C 68,9 H 8,8 N 8,0 % .

Beispiel 7

N-Methyl-D-phenylalanyl-L-prolyl-D-argininaldehyd-sulfat

Stufe 1: N-Benzyloxycarbonyl-N-methyl-D-phenylalanyl-L-prolyl-$N^{\omega}$-benzyloxycarbonyl-D-arginin-lactam

4,3 g (0,011 Mol) $N^{\alpha}$-t-Butyloxycarbonyl-$N^{\omega}$-benzyloxycarbonyl-D-arginin-lactamund 5,09 g (0,01 Mol) N-Benzyloxycarbonyl-N-methyl-D-phenylalanyl-L-prolin-Cyclohexylammoniumsalz (Beispiel 1, Stufe C) werden gemäss dem Verfahren von Beispiel 1, Stufe 1 bei Anwendung von proportionalen Mengen von Reagenzien und Lösungsmitteln umgesetzt bzw. kondensiert.
Ausbeute: 4,5 g (65 %)
$[\alpha]_D^{20}$ = +28,3°(c = 1, Tetrahydrofuran)
$R_F^2$ = 0,55 - 0,65 (Äthylacetat), $R_F$ = 0,7 - 0,8 (D,L,L-Form)

Analyse für $C_{37}H_{42}O_7N_6$ (682.75)

| | | | | | | |
|---|---|---|---|---|---|---|
| berechnet: | C | 65,08 | H | 6,20 | N | 12,31 % |
| gefunden: | C | 65,2 | H | 6,3 | N | 12,2 % . |

Stufe 2: N-Benzyloxycarbonyl-N-methyl-D-phenylalanyl-L-prolyl-$N^{\omega}$-benzyloxycarbonyl-D-argininaldehyd

3,41 g (0,005 Mol) N-Benzyloxycarbonyl-N-methyl-D-phenylalanyl-L-prolyl-$N^{\omega}$-benzyloxycarbonyl-L-arginin-lactam (Beispiel 7, Stufe 1) werden gemäss dem Verfahren von Beispiel 1, Stufe 2 bei Anwendung von proportionalen Mengen von Reagenzien und Lösungsmitteln reduziert.
Ausbeute: 2,7 g (70 %) eines Produktes, das gemäss der Elementaranalyse 1 Mol Cyclohexan enthält.
$R_F^3$ = 0,57 - 0,67
$[\alpha]_D^{20}$ = + 45,5° (c = 1, Tetrahydrofuran)

Analyse für $C_{37}H_{44}O_7N_6 \cdot C_6H_{12}$ (768,93)

| | | | | | | |
|---|---|---|---|---|---|---|
| berechnet: | C | 67,16 | H | 7,34 | N | 10,93 % |
| gefunden: | C | 66,8 | H | 7,1 | N | 10,8 % . |

Stufe 3: N-Methyl-D-phenylalanyl-L-prolyl-D-argininaldehyd-sulfat

2,31 g (0,003 Mol) N-Benzyloxycarbonyl-N-methyl-D-phenylalanyl-L-prolyl-$N^{\omega}$-benzyloxycarbonyl-D-argininaldehyd (Beispiel 7, Stufe 2) werden gemäss dem Verfahren von Beispiel 1, Stufe 3 bei Anwendung von proportionalen Mengen von Reagenzien und Lösungsmitteln hydriert.
Ausbeute: 1,24 g (75 %)
$R_F^6$ = 0,39 - 0,47
$[\alpha]_D^{20}$ = -75° (c = 1, Wasser) .
Die Ausgangssubstanz $N^{\alpha}$-t-Butyloxycarbonyl-$N^{\omega}$-benzyloxycarbonyl-D-arginin-lactam kann gemäss folgendem Verfahren hergestellt werden:

5,97 g (0,14 Mol) $N^{\alpha}$-t-Butyloxycarbonyl-$N^{\omega}$-benzyloxycarbonyl-D-arginin-hydrat, das gemäss dem Verfahren von Beispiel 1, Stufe D hergestellt wird, mit dem Unterschied, dass t-Butyloxycarbonyl-D-argininhydrochlorid als Ausgangsstoff angewendet wird, wird gemäss der Methode von Beispiel 1, Stufe E bei Anwendung von proportionalen Mengen von Reagenzien und Lösungsmitteln umgesetzt.
Ausbeute: 4,0 g (73 %)
Schmelzpunkt: 155 - 156°C
$[\alpha]_D^{20}$ = + 24° (c = 1, Tetrahydrofuran) .

## Beispiel 8

### N-Methyl-D-phenylalanyl-L-prolyl-DL-argininaldehyd-sulfat

Stufe 1: N-Benzyloxycarbonyl-N-methyl-D-phenylalanyl-L-prolyl-$N^{\omega}$-benzyloxycarbonyl-DL-arginin-lactam

4,3 g (0,011 Mol) $N^{\alpha}$-t-Butyloxycarbonyl-$N^{\omega}$-benzyloxycarbonyl-DL-arginin-lactam und 5,09 (0,010 Mol) N-Benzyloxycarbonyl-N-methyl-D-phenylalanyl-L-prolin-Cyclohexylammoniumsalz (Beispiel 1, Stufe C) werden gemäss dem Verfahren von Beispiel 1, Stufe 1 bei Anwendung von proportionalen Mengen von Reagenzien und Lösungsmitteln umgesetzt.
Ausbeute: 4,0 g (60 %)
$[\alpha]_D^{20}$ = + 21° (c = 1, Tetrahydrofuran)
$R_F$ = 0,55 - 0,65 (D,L,D-Form) und $R_F$ = 0,7 - 0,8 (D,L,L-Form) (Äthylacetat).

Stufe 2: N-Benzyloxycarbonyl-N-methyl-D-phenylalanyl-L-prolyl-$N^{\omega}$-benzyloxycarbonyl-DL-argininaldehyd

3,41 g (0,005 Mol) N-Benzyloxycarbonyl-N-methyl-D-phenylalanyl-L-prolyl-$N^{\omega}$-benzyloxycarbonyl-DL-arginin-lactam (Beispiel 8, Stufe 1) werden gemäss dem Verfahren von Beispiel 1, Stufe 2 bei Anwendung von proportionalen Mengen von Reagenzien und Lösungsmitteln umgesetzt.
Ausbeute: 2,9 g (75 %) eines Produktes, das 1 Mol Cyclohexan enthält.
$R_F^3$ = 0,52 - 0,67
$[\alpha]_D^{20}$ = + 32,5° (c = 1, Tetrahydrofuran) .

Stufe 3: N-Methyl-D-phenylalanyl-L-prolyl-DL-argininaldehyd-sulfat

2,31 g (0,003 Mol) N-Benzyloxycarbonyl-N-methyl-D-phenylalanyl-L-prolyl-$N^{\omega}$-benzyloxycarbonyl-DL-argininaldehyd (Beispiel 8, Stufe 2) werden gemäss dem Verfahren von Beispiel 1, Stufe 3 bei Anwendung von proportionalen Mengen von Reagenzien und Lösungsmitteln umgesetzt.
Ausbeute: 1,15 g (70 %)
$R_F^6$ = 0,39 - 0,47
$[\alpha]_D^{20}$ = - 109° (c = 1, Wasser) .

Beispiel 9

Herstellung eines Arzneimittelpräparates

Das für 6 und 12 stündige Infusion geeignete 2-Ampullen Präparat wird folgendermassen hergestellt:
N-Methyl-D-phenylalanyl-L-prolyl-L-argininaldehyd-sulfat (420 - 840 mg) und Humanalbumin (40 - 80mg) werden zusammen lyophilisiert. Der Inhalt der lyophilisierten Ampullen wird unmittelbar vor Anwendung in steriler, keimfreier, physiologischer Salzlösung der anderen Ampulle gelöst (100-200 ml).

**Patentansprüche**

**Patentansprüche für folgende Vertragsstaaten: BE, CH, DE, FR, GB, IT, LI, LU, NL, SE**

**1.** Tripeptidyl-aldehyde der allgemeinen Formel

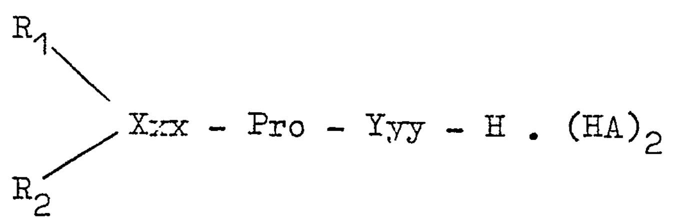

I ,

worin

| | |
|---|---|
| $R_1$ | für ein Wasserstoffatom oder einen Alkylrest mit 1 bis 6 Kohlenstoffatom(en) steht, |
| $R_2$ | einen Alkylrest mit 1 bis 6 Kohlenstoffatom(en) bedeutet, |
| Xxx | einen Rest von D-Phenylalanin oder einen Rest einer D-α-Aminosäure, die in der Seitenkette einen Alkylrest mit 1 bis 4 Kohlenstoffatom(en) hat, darstellt, |
| Pro | für einen Rest von L-Prolin steht, |
| Yyy | einen Rest von L-, D- oder DL-Arginin bedeutet und |
| A | einen Acylrest darstellt, |

wobei $R_1$ und $R_2$ an die Aminogruppe des Restes der Aminosäure, für die Xxx stehen kann, gebunden ist.

**2.** Tripeptidyl-aldehyde nach Anspruch 1, dadurch gekennzeichnet, daß der beziehungsweise die Alkylrest(e), für den beziehungsweise die $R_1$ und/oder $R_2$ steht beziehungsweise stehen kann beziehungsweise können, [ein] solche[r] mit 1 bis 4, insbesondere 1 oder 2, Kohlenstoffatom(en) ist beziehungsweise sind, wobei besonders bevorzugt $R_1$ für ein Wasserstoffatom oder einen Methylrest steht und $R_2$ einen Methylrest bedeutet.

**3.** Tripeptidyl-aldehyde nach Anspruch 1 oder 2, dadurch gekennzeichnet, daß Xxx einen Rest von D-Phenylalanin darstellt.

**4.** Tripeptidyl-aldehyde nach Anspruch 1 oder 2, dadurch gekennzeichnet, daß der Alkylrest der Seitenkette des Restes der D-α-Aminosäure, den Xxx darstellen kann, ein solcher mit 3 oder 4, insbesondere 4, Kohlenstoffatomen ist, wobei für Xxx als Rest der D-α-Aminosäure ein solcher von D-Alloisoleucin, D-Isoleucin, D-Norleucin, D-Valin oder D-Norvalin besonders bevorzugt ist.

**5.** Tripeptidyl-aldehyde nach Anspruch 1 bis 4, dadurch gekennzeichnet, daß Yyy einen Rest von L-Arginin bedeutet.

**6.** Tripeptidyl-aldehyde nach Anspruch 1 bis 5, dadurch gekennzeichnet, daß der Acylrest, für welchen A steht, ein Salzsäure-, Schwefelsäure-, Phosphorsäure-, Salpetersäure-, Weinsäure-, Essigsäure-, Citronensäure-, Apfelsäure-, Milchsäure-, Fumarsäure, Benzoesäure-, Glykolsäure-, Gluconsäure-, Gulonsäure-, Bernsteinsäure- oder Arylsulfonsäurerest ist.

**7.** Die Tripeptidyl-aldehyde N-(Methyl)-D-phenylalanyl-L-prolyl-L-argininaldehyd-sulfat, N,N-Di-(methyl)-D-phenylalanyl-L-prolyl-L-argininaldehyd-sulfat, N-(Äthyl)-D-phenylalanyl-L-prolyl-L-argininaldehyd-sulfat, N-(Isobutyl)-D-phenylalanyl-L-prolyl-L-argininaldehyd-sulfat, N-(Methyl)-D-alloisoleucyl-L-prolyl-L-argininaldehyd-sulfat und N-(n-Hexyl)-D-phenylalanyl-L-prolyl-L-argininaldehyd-sulfat.

**8.** Verfahren zur Herstellung der Verbindungen nach Anspruch 1 bis 7, dadurch gekennzeichnet, daß man das an der Guanidinogruppe geschützte Arginin-lactam mittels einer in der Peptidchemie bekannten Verfahrensweise mit einem an seiner endständigen Aminogruppe eine Schutzgruppe aufweisenden N-(Monoalkyl)-Xxx-L-prolin-Dipeptid oder einem N,N-Di-(alkyl)-Xxx-L-prolin-Dipeptid, der allgemeinen Formel

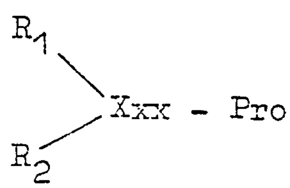

II ,

worin $R_1$ , $R_2$ und Xxx die in den Ansprüchen 1 bis 5 angegebenen Bedeutungen hat, kondensiert, das erhaltene geschützte Tripeptidyl-lactam zum entsprechenden geschützten Tripeptidylaldehyd reduziert und nach Abspalten der Schutzgruppe(n) den N-(Alkyl- beziehungsweise N,N-Di-(alkyl)-tripeptidyl-aldehyd in Salzform isoliert.

**9.** Arzneimittel, gekennzeichnet durch einen Gehalt an 1 oder mehr Verbindung(en) nach Anspruch 1 bis 7 als Wirkstoff(en), zweckmäßig zusammen mit 1 oder mehr üblichen pharmazeutischen Konfektionierungsmittel(n).

**10.** N-(Monoalkyl)- und N,N-Di-(alkyl)-Xxx-L-prolin-Dipeptide der allgemeinen Formel

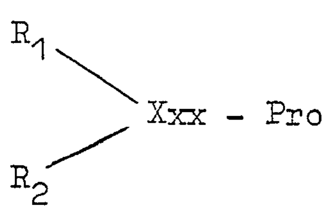

II ,

worin

$R_1$ für ein Wasserstoffatom oder einen Alkylrest mit 1 bis 6 Kohlenstoffatom(en) steht,

$R_2$ einen Alkylrest mit 1 bis 6 Kohlenstoffatom(en) bedeutet und

Xxx einen Rest von D-Phenylalanin oder einen Rest einer D-α-Aminosäure, die in der Seitenkette einen Alkylrest mit 1 bis 4 Kohlenstoffatom(en) hat, darstellt.

**Patentansprüche für folgenden Vertragsstaat: AT**

1. Verfahren zur Herstellung von Tripeptidyl-aldehyden der allgemeinen Formel

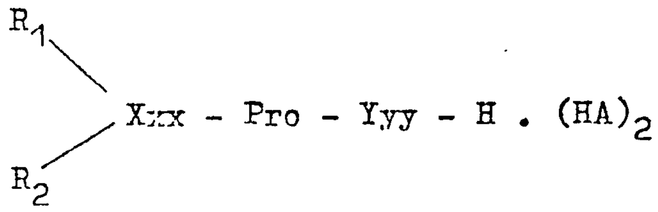

I ,

worin

$R_1$ für ein Wasserstoffatom oder einen Alkylrest mit 1 bis 6 Kohlenstoffatom(en) steht,
$R_2$ einen Alkylrest mit 1 bis 6 Kohlenstoffatom(en) bedeutet,
Xxx einen Rest von D-Phenylalanin oder einen Rest einer D-α-Aminosäure, die in der Seitenkette einen Alkylrest mit 1 bis 4 Kohlenstoffatom(en) hat, darstellt,
Pro für einen Rest von L-Prolin steht,
Yyy einen Rest von L-, D- oder DL-Arginin bedeutet und
A einen Acylrest darstellt,

wobei $R_1$ und $R_2$ an die Aminogruppe des Restes der Aminosäure, für die Xxx stehen kann, gebunden ist,
dadurch gekennzeichnet, daß man
das an der Guanidinogruppe geschützte Arginin-lactam mittels einer in der Peptidchemie bekannten Verfahrensweise mit einem an seiner endständigen Aminogruppe eine Schutzgruppe aufweisenden N-(Monoalkyl)-Xxx-L-prolin-Dipeptid oder einem N,N-Di-(alkyl)-Xxx-L-prolin-Dipeptid der allgemeinen Formel

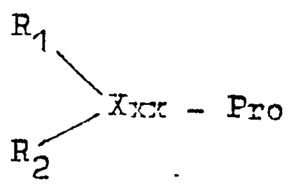

II ,

worin $R_1$ , $R_2$ und Xxx die oben angegebenen Bedeutungen hat, kondensiert, das erhaltene geschützte Tripetidyl-lactam zum entsprechenden geschützten Tripeptidyl-aldehyd reduziert und nach Abspalten der Schutzgruppe(n) den N-(Alkyl)- beziehungsweise N,N-Di-(alkyl)-tripeptidyl-aldehyd in Salzform isoliert.

2. Verfahren nach Anspruch 1, dadurch gekennzeichnet, daß man als an seiner endständigen Aminogruppe eine Schutzgruppe aufweisendes N-(Monoalkyl)-Xxx-L-prolin-Dipeptid oder N,N-Di-(alkyl)-Xxx-L-prolin-Dipeptid der allgemeinen Formel II ein solches, bei welchem der beziehungsweise die Alkylrest(e), für den beziehungsweise die $R_1$ und/oder $R_2$ steht beziehungsweise stehen kann beziehungsweise können, [ein] solche[r] mit 1 bis 4, insbesondere 1 oder 2, Kohlenstoffatom(en) ist beziehungsweise sind, wobei besonders bevorzugt $R_1$ für ein Wasserstoffatom oder einen Methylrest steht und $R_2$ einen Methylrest bedeutet, verwendet.

3. Verfahren nach Anspruch 1 oder 2, dadurch gekennzeichnet, daß man als an seiner endständigen Aminogruppe eine Schutzgruppe aufweisendes N-(Monoalkyl)-Xxx-L-prolin-Dipeptid oder N,N-Di-(alkyl)-Xxx-L-prolin-Dipeptid der allgemeinen Formel II ein solches, bei welchem Xxx einen Rest von D-Phenylalanin darstellt, verwendet.

4. Verfahren nach Anspruch 1 oder 2, dadurch gekennzeichnet, daß man als an seiner endständigen Aminogruppe eine Schutzgruppe aufweisendes N-(Monoalkyl)-Xxx-L-prolin-Dipeptid oder N,N-Di-(alkyl)-Xxx-L-prolin-Dipeptid der allgemeinen Formel II ein solches, bei welchem der Alkylrest der Seitenkette des Restes der D-α-Aminosäure, den Xxx darstellen kann, ein solcher mit 3 oder 4, insbesondere 4, Kohlenstoffatomen ist, wobei für Xxx als Rest der D-α-Aminosäure ein solcher von D-Alloisoleucin, D-Isoleucin, D-Norleucin, D-Valin oder D-Norvalin besonders bevorzugt ist, verwendet.

5. Verfahren nach Anspruch 1 bis 4, dadurch gekennzeichnet, daß man als an seiner endständigen Aminogruppe eine Schutzgruppe aufweisendes N-(Monoalkyl)-Xxx-L-prolin-Dipeptid oder N,N-Di-(alkyl)-Xxx-L-prolin-Dipeptid der allgemeinen Formel II ein solches, bei welchem Yyy einen Rest von L-Arginin bedeutet, verwendet.

6. Verfahren nach Anspruch 1 bis 5, dadurch gekennzeichnet, daß man als an seiner endständigen Aminogruppe eine Schutzgruppe aufweisendes N-(Monoalkyl)-Xxx-L-prolin-Dipeptid oder N,N-Di-(alkyl)-Xxx-L-prolin-Dipeptid der allgemeinen Formel II ein solches, bei welchem der Acylrest, für welchen A steht, ein Salzsäure-, Schwefelsäure-, Phosphorsäure-, Salpetersäure-, Weinsäure-, Essigsäure-, Citronensäure-, Apfelsäure-, Milchsäure-, Fumarsäure-, Benzoesäure-, Glykolsäure-, Gluconsäure-, Gulonsäure-, Bernsteinsäure- oder Arylsulfonsäurerest ist, verwendet.

7. Verfahren nach Anspruch 1 bis 6, dadurch gekennzeichnet, daß man als Tripeptidyl-aldehyde N-(Methyl)-D-phenylalanyl-L-prolyl-L-argininaldehyd-sulfat, N,N-Di-(methyl)-D-phenylalanyl-L-prolyl-L-argininaldehyd-sulfat, N-(Äthyl)-D-phenylalanyl-L-prolyl-L-argininaldehyd-sulfat, N-(Isobutyl)-D-phenylalanyl-L-prolyl-L-argininaldehyd-sulfat, N-(Methyl)-D-alloisoleucyl-L-prolyl-L-argininaldehyd-sulfat und N-(n-Hexyl)-D-phenylalanyl-L-prolyl-L-argininaldehyd-sulfat herstellt.

## Claims

**Claims for the following Contracting States: BE, CH, DE, FR, GB, IT, LI, LU, NL, SE**

1. Tripeptidylaldehydes of the general formula

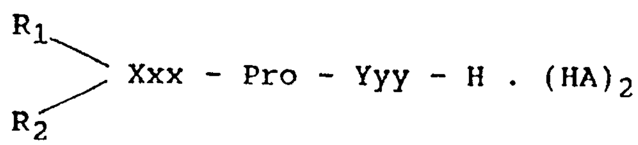

I,

wherein

| | |
|---|---|
| $R_1$ | is a hydrogen atom or an alkyl radical having from 1 to 6 carbon atoms, |
| $R_2$ | is an alkyl radical having from 1 to 6 carbon atoms, |
| Xxx | is a residue of D-phenylalanine or a residue of a D-α-amino acid having in the side chain an alkyl radical having from 1 to 4 carbon atoms, |
| Pro | is a residue of L-proline, |
| Yyy | is a residue of L-, D- or DL-arginine, and |
| A | is an acyl radical, |

$R_1$ and $R_2$ being bonded to the amino group of the residue of the amino acid which Xxx may represent.

2. Tripeptidylaldehydes according to claim 1, characterised in that the alkyl radical(s) which $R_1$ and/or $R_2$ may represent is(are) such (a) radical(s) having from 1 to 4, especially 1 or 2, carbon atoms, and especially preferably $R_1$ is a hydrogen atom or a methyl radical and $R_2$ is a methyl radical.

3. Tripeptidylaldehydes according to claim 1 or 2, characterised in that Xxx is a residue of D-phenylalanine.

4. Tripeptidylaldehydes according to claim 1 or 2, characterised in that the alkyl radical of the side chain of the residue of the D-α-amino acid which Xxx may represent is such a radical having 3 or 4, especially 4, carbon atoms, Xxx as a residue of the D-α-amino acid being especially preferably a residue of D-alloisoleucine, D-isoleucine, D-norleucine, D-valine or D-norvaline.

5. Tripeptidylaldehydes according to claims 1 to 4, characterised in that Yyy is a residue of L-arginine.

6. Tripeptidylaldehydes according to claims 1 to 5, characterised in that the acyl radical represented by A is a hydrochloric acid, sulphuric acid, phosphoric acid, nitric acid, tartaric acid, acetic acid, citric acid, malic acid, lactic acid, fumaric acid, benzoic acid, glycolic acid, gluconic acid, gulonic acid, succinic acid or arylsulphonic acid radical.

7. The tripeptidylaldehydes N-(methyl)-D-phenylalanyl-L-prolyl-L-arginine aldehyde sulphate, N,N-di-(methyl)-D-phenylalanyl-L-prolyl-L-argininealdehyde sulphate, N-(ethyl)-D-phenylalanyl-L-prolyl-L-arginine aldehyde sulphate, N-(isobutyl)-D-phenylalanyl-L-prolyl-L-arginine aldehyde sulphate, N-(methyl)-D-alloisoleucyl-L-prolyl-L-arginine aldehyde sulphate and N-(n-hexyl)-D-phenylalanyl-L-prolyl-L-arginine aldehyde sulphate.

8. A process for the preparation of the compounds according to claims 1 to 7, characterised in that argininelactam protected at the guanidino group is condensed using a procedure known in peptide chemistry with an N-(monoalkyl)-Xxx-L-proline dipeptide having a protecting group at its terminal amino group, or an N,N-di-(alkyl)-Xxx-L-proline dipeptide, of the general formula

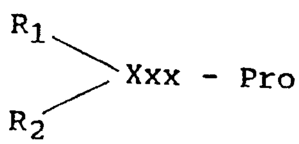

II,

wherein $R_1$, $R_2$ and Xxx have the meanings given in claims 1 to 5, the resulting protected tripeptidyllactam is reduced to the corresponding protected tripeptidylaldehyde and after removal of the protecting group(s) the N-(alkyl)- or N,N-di-(alkyl)-tripeptidylaldehyde is isolated in salt form.

9. Medicaments, characterised by a content of one or more compounds according to claims 1 to 7 as active ingredient(s), advantageously together with one or more customary pharmaceutical confectioning agents.

10. N-(monoalkyl)- and N,N-di-(alkyl)-Xxx-L-proline dipeptides of the general formula

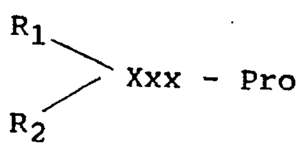

II,

wherein

$R_1$ is a hydrogen atom or an alkyl radical having from 1 to 6 carbon atoms,

$R_2$ is an alkyl radical having from 1 to 6 carbon atoms, and

Xxx is a residue of D-phenylalanine or a residue of a D-$\alpha$-amino acid having in the side chain an alkyl radical having from 1 to 4 carbon atoms.

**Claims for the following Contracting State: AT**

1. A process for the preparation of tripeptidylaldehydes of the general formula

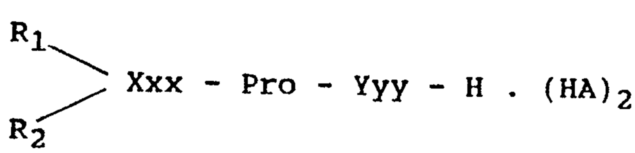

I,

wherein

- $R_1$ is a hydrogen atom or an alkyl radical having from 1 to 6 carbon atoms,
- $R_2$ is an alkyl radical having from 1 to 6 carbon atoms,
- Xxx is a residue of D-phenylalanine or a residue of a D-$\alpha$-amino acid having in the side chain an alkyl radical having from 1 to 4 carbon atoms,
- Pro is a residue of L-proline,
- Yyy is a residue of L-, D- or DL-arginine, and
- A is an acyl radical,

$R_1$ and $R_2$ being bonded to the amino group of the residue of the amino acid which Xxx may represent, characterised in that

argininelactam protected at the guanidino group is condensed using a procedure known in peptide chemistry with an N-(monoalkyl)-Xxx-L-proline dipeptide having a protecting group at its terminal amino group, or an N,N-di-(alkyl)-Xxx-L-proline dipeptide, of the general formula

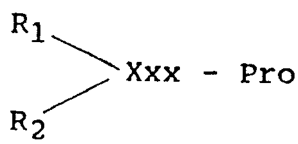

II,

wherein $R_1$, $R_2$ and Xxx have the meanings given in claims 1 to 5, the resulting protected tripeptidyllactam is reduced to the corresponding protected tripeptidylaldehyde and after removal of the protecting group(s) the N-(alkyl)- or N,N-di-(alkyl)-tripeptidylaldehyde is isolated in salt form.

2. A process according to claim 1 characterised in that tripeptidylaldehydes are prepared, wherein the alkyl radical(s) which $R_1$ and/or $R_2$ may represent is(are) such (a) radical(s) having from 1 to 4, especially 1 or 2, carbon atoms, and especially preferably $R_1$ is a hydrogen atom or a methyl radical and $R_2$ is a methyl radical.

3. A process according to claim 1 or 2 characterised in that tripeptidylaldehydes are prepared, wherein Xxx is a residue of D-phenyl-alanine.

4. A process according to claim 1 or 2, characterised in that tripeptidylaldehydes are prepared, wherein the alkyl radical of the side chain of the residue of the D-$\alpha$-amino acid which Xxx may represent is such a radical having 3 or 4, especially 4, carbon atoms, Xxx as a residue of the D-$\alpha$-amino acid being especially preferably a residue of D-alloisoleucine, D-isoleucine, D-norleucine, D-valine or D-norvaline.

5. A process according to claims 1 to 4, characterised in that tripeptidylaldehydes are prepared, wherein Vyy is a residue of L-arginine.

6. A process according to claims 1 to 5, characterised in that tripeptidylaldehydes are prepared wherein the acyl radical represented by A is a hydrochloric acid, sulphuric acid, phosphoric acid, nitric acid, tartaric acid, acetic acid, citric acid, malic acid, lactic acid, fumaric acid, benzoic acid, glycolic acid, gluconic acid, gulonic acid, succinic acid or arylsulphonic acid radical.

7. A process according to claims 1 to 6, characterised in that tripeptidylaldehydes N-(methyl)-D-phenylalanyl-L-prolyl-L-argininealdehyde sulphate, N,N-di-(methyl)-D-phenylalanyl-L-prolyl-L-argininealdehyde sulphate, N-(ethyl)-D-phenylalanyl-L-prolyl-L-arginine aldehyde sulphate, N-(isobutyl)-D-phenylalanyl-L-prolyl-L-arginine aldehyde sulphate, N-(methyl)-D-alloisoleucyl-L-prolyl-L-arginine aldehyde sulphate and N-(n-hexyl)-D-phenylalanyl-L-prolyl-L-arginine aldehyde sulphate are prepared.

**Revendications**

**Revendications pour les Etats contractants suivants: BE, CH, DE, FR, GB, IT, LI, LU, NL, SE**

1. Tripeptidyl-aldéhyde de formule générale :

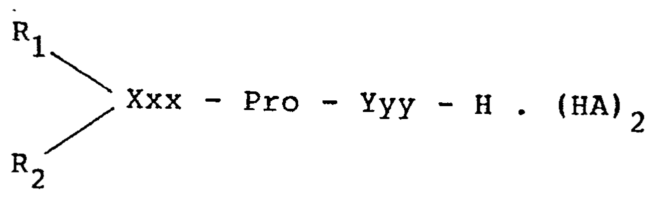

I

dans laquelle :

$R_1$ représente un atome d'hydrogène ou un reste alkyle de 1 à 6 atomes de carbone,

$R_2$ signifie un reste alkyle de 1 à 6 atomes de carbone,

Xxx représente un reste de D-phénylalanine ou bien un reste d'acide D-α-aminé, comportant dans la chaîne secondaire un reste alkyle de 1 à 4 atomes de carbone,

Pro représente un reste de L-proline,

Yyy signifie un reste de L-, D- ou DL-arginine, et

A représente un reste acyle,

$R_1$ et $R_2$ étant liés au groupe amino du reste de l'acide amino, lequel peut représenter Xxx.

**2.** Tripeptidyl-aldéhyde selon la revendication 1, caractérisé en ce que le ou les restes alkyles, pour lequel ou pour lesquels $R_1$ et/ou $R_2$ est ou peut être ou peuvent être, est ou sont un ou des groupes d'atomes de carbone de 1 à 4 en particulier de 1 ou 2, et où en particulier $R_1$ représente un atome d'hydrogène ou un reste méthyle et $R_2$ signifie un reste méthyle.

**3.** Tripeptidyl-aldéhyde selon la revendication 1 ou 2, caractérisé en ce que Xxx représente un reste de D-phénylalanine.

**4.** Tripeptidyl-aldéhyde selon la revendication 1 ou 2, caractérisé en ce que le reste alkyle de la chaîne secondaire du reste d'acide D-α-aminé, qui représente Xxx, est un groupe de 3 ou 4 atomes de carbone, en particulier de 4 atomes, où comme reste de l'acide D-α-aminé pour Xxx on préfère en particulier celui de la D-alloisoleucine, de la D-isoleucine, de la D-norleucine, de la D-valine ou la D-norvaline.

**5.** Tripeptidyl-aldéhyde selon l'une des revendications 1 à 4, caractérisé en ce que Yyy signifie un reste de L-arginine.

**6.** Tripeptidyl-aldéhyde selon l'une des revendications 1 à 5, caractérisé en ce que le reste acyle que représente A est un reste d'acide chlorhydrique, sulfonique, phosphorique, nitrique, tartrique, acétique, citrique, malique, lactique, fumarique, benzoïque, glycolique, glyconique, succinique ou arylsulfonique.

**7.** Tripeptidyl-aldéhyde de N-(méthyl)-D-phénylalanyl-L-prolyl-L-argininaldéhyde-sulfate, de N-N-di-(méthyl)-D-phénylalanyl-L-prolyl-L-argininaldéhyde-sulfate, de N-(éthyl)-D-phénylalanyl-L-prolyl-L-argininaldéhydesulfate, de N-(isobutyl)-D-phénylalanyl-L-prolyl-L-argininaldéhyde-sulfate, de N-(méthyl)-D-alloisoleucyl-L-prolyl-L-argininaldéhyde-sulfate et de N-(n-hexyl)-D-phénylalanyl-L-prolyl-L-argininaldéhyde-sulfate.

**8.** Procédé pour la fabrication des composés selon l'une des revendications 1 à 7, caractérisé en ce qu'on condense l'argininlactame, protégé au niveau du groupe aminé, au moyen d'un procédé connu dans la chimie des peptides avec un n-(monoalkyl)-Xxx-L-prolin-dipeptide ou un N,N-di(alkyl)-Xxx-L-prolin-dipeptide protégé au niveau de son groupe amino terminal, un groupe de protection de formule générale :

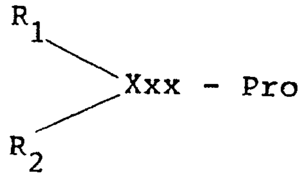

II

dans laquelle $R_1$ , $R_2$ et Xxx ont les significations indiquées dans les revendications 1 à 5, on réduit le tripeptidyl-lactame protégé obtenu en tripeptidyl-aldéhyde protégé correspondant et on isole après séparation du ou des groupes de protection le N-(alkyl- ou N,N-di-(alkyl)-tripeptidyl-aldéhyde sous forme de sel.

**9.** Médicament, caractérisé en ce qu'il renferme comme principe actif un ou plusieurs composés selon les revendications 1 à 7, avec de manière convenable un ou plusieurs moyens de préparation pharmaceutiques usuels.

**10.** N-(monoalkyl)- et N,N-di-(alkyl)-Xxx-L-prolin-dipeptide de formule générale :

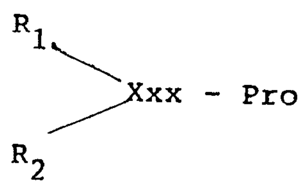

II

dans laquelle
- $R_1$ représente un atome d'hydrogène ou un reste alkyle de 1 à 6 atomes de carbone,
- $R_2$ signifie un reste alkyle de 1 à 6 atomes de carbone,
- Xxx représente un reste de D-phénylalanine ou un reste d'un acide D-$\alpha$-amino, qui possède dans la chaîne latérale un reste alkyle ayant 1 à 4 atomes de carbone.

**Revendications pour l'Etat contractant suivant: AT**

**1.** Procédé de préparation des tripeptidyl-aldéhydes de formule générale :

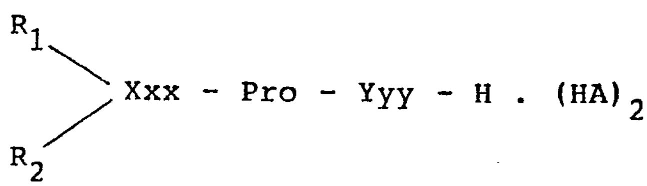

I

dans laquelle :
- $R_1$ représente un atome d'hydrogène ou un reste alkyle de 1 à 6 atomes de carbone,
- $R_2$ signifie un reste alkyle de 1 à 6 atomes de carbone,
- Xxx représente un reste de D-phénylalanine ou bien un reste d'acide D-$\alpha$-aminé, comportant dans la chaîne secondaire un reste alkyle de 1 à 4 atomes de carbone,
- Pro représente un reste de L-proline,
- Yyy signifie un reste de L-, D- ou DL-arginine, et
- A représente un reste acyle,

$R_1$ et $R_2$ étant liés au groupe amino du reste de l'acide amino, lequel peut représenter Xxx, caractérisé en ce qu'on condense l'argininlactame, protégé au niveau du groupe aminé, au moyen d'un procédé connu dans la chimie des peptides avec un n-(monoalkyl)-Xxx-L-prolin-dipeptide ou un N,N-di(alkyl)-Xxx-L-prolin-dipeptide protégé au niveau de son groupe amino terminal, un groupe de protection de

formule générale :

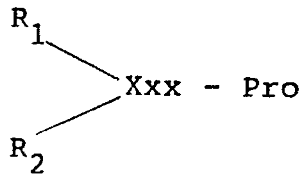

II

dans laquelle $R_1$ , $R_2$ et Xxx ont les significations indiquées dans les revendications 1 à 5, on réduit le tripeptidyl-lactame protégé obtenu en tripeptidyl-aldéhyde protégé correspondant et on isole après séparation du ou des groupes de protection le N-(alkyl- ou N,N-di-(alkyl)-tripeptidyl-aldéhyde sous forme de sel.

**2.** Procédé selon la revendication 1, caractérisé en ce que les tripeptidyl-aldéhydes sont preparés, en ce que le ou les restes alkyles, pour lequel ou pour lesquels $R_1$ et/ou $R_2$ est ou peut être ou peuvent être, est ou sont un ou des groupes d'atomes de carbone de 1 à 4 en particulier de 1 ou 2, et ou en particulier $R_1$ représente un atome d'hydrogène ou un reste méthyle et $R_2$ signifie un reste méthyle.

**3.** Procédé selon la revendication 1 ou 2, caractérisé en ce que tripeptidyl-aldéhydes sont preparés, en ce que Xxx représente un reste de D-phénylalanine.

**4.** Procédé selon la revendication 1 ou 2, caractérisé en ce que les tripeptidyl-aldéhydes sont preparés, en ce que le reste alkyle de la chaîne secondaire du reste d'acide D-$\alpha$-aminé, qui représente Xxx, est un groupe de 3 ou 4 atomes de carbone, en particulier de 4 atomes, où comme reste de l'acide D-$\alpha$-aminé pour Xxx on préfère en particulier celui de la D-alloisoleucine, de la D-isoleucine, de la D-norleucine, de la D-valine ou la D-norvaline.

**5.** Procédé selon la revendications 1 à 4, caractérisé en ce les tripeptidyl-aldéhydes sont preparés, en ce que Yyy signifie un reste de L-arginine.

**6.** Procédé selon la revendications 1 à 5, caractérisé en ce que les tripeptidyl-aldéhydes sont preparés, en ce que le reste acyle que représente A est un reste d'acide chlorhydrique, sulfonique, phosphorique, nitrique, tartrique, acétique, citrique, malique, lactique, fumarique, benzoîque, glycolique, glyconique, succinique ou arylsulfonique.

**7.** Procédé selon la revendications 1 à 6, caractérisé en ce que les tripeptidyl-aldéhydes de N-(methyl)-D-phénylalanyl-L-prolyl-L-argininaldéhyde-sulfate, de N-N-di-(méthyl)-D-phénylalanyl-L-prolyl-L-argininaldéhyde-sulfate, de N-(éthyl)-D-phénylalanyl-L-prolyl-L-argininaldéhyde-sulfate, de N-(isobutyl)-D-phénylalanyl-L-prolyl-L-argininaldéhyde-sulfate, de N-(méthyl)-D-alloisoleucyl-L-prolyl-L-argininaldéhyde-sulfate et de N-(n-hexyl)-D-phénylalanyl-L-prolyl-L-argininaldéhyde-sulfate sont preparés.